# EUROPEAN PATENT APPLICATION

(11) **EP 2 474 611 A1**
(43) Date of publication of application: **11.07.2012**
(21) Application number: 10813526.0
(22) Date of filing: 03.09.2010
(51) Int. Cl.: C12N 15/09, C12N 5/10

(54) **ENHANCER FOR PROMOTER, AND USE THEREOF**

(30) Priority: 04.09.2009 JP 2009205365
(71) Applicant: National Institute of Biomedical Innovation, Ibaraki-shi Osaka 567-0085 (JP); The Research Foundation for Microbial Diseases of Osaka University, Suita-shi, Osaka 565-0871 (JP)
(72) Inventor: MATSUURA, Masaaki, Kanonji-shi Kagawa 768-0061 (JP); TAKEMOTO, Masaya, Ibaraki-shi Osaka 567-0085 (JP); KOSHIZUKA, Tetsuo, Ibaraki-shi Osaka 567-0085 (JP); YAMANISHI, Koichi, Ibaraki-shi Osaka 567-0085 (JP); MORI, Yasuko, Ibaraki-shi Osaka 567-0085 (JP)
(74) Representative: Train, Matthew
(86) International application number: PCT/JP2010/005447
(87) International publication number: WO 2011/027575

(57) **Abstract**

Disclosed is an enhancer for a viral promoter such as a promoter that can induce expression selectively and strongly in immunocompetent cells (e.g., lymphocytes) or blood cells. It is found unexpectedly that an intron has the above-mentioned enhancer activity. Thus, it is found that an enhancer for a promoter, which comprises an intron sequence for a major immediate early gene (MIE) of human herpes virus-6 (HHV-6) (particularly HHV-6B) or a fragment of the intron sequence, has a potent promoter activity.

## Description

### TECHNICAL FIELD

The present invention relates to an enhancer for promoter and the use thereof.

### BACKGROUND ART

There has been a demand for the establishment of a technique for gene therapy on lymphoid cells in order to treat various diseases targeting lymphoid cells, such as leukemia and human immunodeficiency virus (HIV) infection. The present inventors have found out an HHV-6B MIE promoter from human herpesvirus-6 (HHV-6), one kind of herpesvirus, as a promoter for introducing a desired gene into lymphocyte cells, and filed the application thereof (Patent Document 1).

Such a virus promoter can be used alone, but is desired to be combined with an enhancer to enhance its action.

In this regard, with respect to eukaryotic promoters, various introns have been known to have enhancer functions. However, with respect to promoters of viruses such as herpesvirus, the enhancer function of the intron has not been known well.

A CMV promoter has been known to be poor in its activity in lymphoid cells. Even if an enhancer of Patent document 2 is used, it is unknown whether the activity increases in cells other than CHO cells.
Patent Document 1: International Publication No. 2007/029712 pamphlet
Patent Document 2: Japanese PCT National Phase Laid-Open Publication No. 2008-536506

### SUMMARY OF THE INVENTION

### PROBLEMS TO BE SOLVED BY THE INVENTION

The present invention intends to provide an enhancer for a viral promoter such as a promoter that can induce expression selectively and strongly in immunocompetent cells or blood cells, such as lymphocytes and the like.

### SOLUTIONS TO THE PROBLEMS

The present invention has solved the above problem by unexpectedly finding that an intron for a major immediate early gene (MIE) of HHV-6B has such an enhancer activity.

This may be significant in that, even though all introns do not have enhanced expression effects, an intron having such an effect has been found newly. This may be also significant in that, even though most of past studies have been intended for eukaryotes, an intron of HHV-6 is shown by the present inventors (it may be the fist time at least for HHV-6, particularly HHV-6B) and the effect thereof is remarkable.

The CMV promoter has been known to be poor in its actiVity in lymphoid cells. Even if an enhancer of Patent document 2 is used, it is unknown whether the activity increases in cells other than CHO cells. Even if the activity increases in lymphoid cells, such an increase is not always favorable because the original activity is small. In that respect, the 6MIE promoter by itself shows higher activity than the CMV promoter in cultured cells from T-cell lines such as Molt-3, Jurkat, and SupT1. A combination of this 6MIE promoter with the present enhancer causes a further increase in activity. Besides, it is found that such a combination has a promoter activity at least 5 times higher than that of the CMV promoter. Thus, it is expected that a foreign gene can be more effectively introduced into lymphoid cells in a specific manner.

Therefore, the present specification provides the following items.
(1) An enhancer for a promoter, comprising: an intron sequence for a major immediate early gene (MIE) of human herpes virus-6 (HHV-6) (hereinafter, referred to as HHV-6B), or a fragment of the intron sequence.
(2) The enhancer for a promoter according to item 1, wherein the intron sequence includes a sequence (SEQ ID No. 2) of at least -12 to +1292 based on a transcription initiation point of IE1 included in the above 6MIE sequence, or a fragment of the sequence.
(3) The enhancer for a promoter according to item 1 or 2, wherein the sequence includes a sequence (SEQ ID No. 3) of -12 to +262 based on the transcription initiation point of IE1 included in the above 6MIE sequence.
(4) The enhancer for a promoter according to any of items 1 to 3, wherein the sequence is the sequence (SEQ ID No. 3) of - 12 to +262 based on the transcription initiation point of IE1 included in the above 6MIE sequence.
(5) The enhancer for a promoter according to any of items 1 to 4, wherein the promoter includes a sequence (SEQ ID No. 4) of at least -382 to -983 based on the transcription initiation point of IE1 included in the above 6MIE sequence, or a fragment of the sequence.
(6) A promoter with improved activity, comprising: an intron sequence for a major immediate early gene (MIE) of human herpes virus-6 (HHV-6) or a fragment of the sequence; and a promoter.
(7) The promoter with improved activity according to item 6, further comprising: a sequence (SEQ ID No. 2) of at least -12 to +1292 based on a transcription initiation point of IE1 included in the above 6MIE sequence, or a fragment of the sequence; and an MIE promoter.
(8) The promoter with improved activity according to item 7, wherein the MIE promoter includes a sequence (SEQ ID No. 4) of at least -382 to -983 based on the transcription initiation point of IE1 included in the above 6MIE sequence, or a fragment of the sequence.
(9) A construct for enhancing expression of a gene in a cell, comprising: an intron sequence for a major immediate early gene (MIE) of human herpes virus-6 (HHV-6) or a fragment of the sequence; and a promoter.
(10) The construct according to item 9, wherein the construct includes: a sequence (SEQ ID No. 2) of at least -12 to +1292 based on a transcription initiation point of IE1 included in the above 6MIE sequence, or a fragment of the sequence; and an MIE promoter.
(11) The construct according to item 9 or 10, wherein the cell is a cell of T-lymphocyte or adherent line.
(12) The construct according to items 9, 10, or 11, wherein the cell is selected from the group consisting of Molt-3, Jurkat, MRC-5, MeWo, SupT1, and U373.
(13) A method for enhancing expression of a gene in a cell, comprising the steps of:
   1) generating a construct including the enhancer for a promoter as described in any one of items 1 to 5 and a promoter, where the gene is arranged so as to be operatively linked to a sequence;
   2) introducing the construct into the cell; and
   3) culturing the cell under conditions for expressing the gene.
(14) Use of a sequence (SEQ ID No. 2) of at least -12 to +1292 based on a transcription initiation point of IE1 included in the 6MIE sequence, or a fragment of the sequence, as an enhancer for a promoter.

Hereinafter, preferred embodiments of the present invention are presented. It should be understood that those skilled in the art would appropriately perform the embodiments thereof based on the description of the present invention and the well-known and routinely used technique in the art, and the functions and effects attained by the present invention should be readily understood.

### ADVANTAGEOUS EFFECTS OF INVENTION

The present invention has provided enhancers for promoters that selectively induce the expression of a protein in immune cells such as T lymphocytes. The use of enhancers for promoters of the present invention has provided methods and pharmaceutical agents for effectively preventing or treating immunological diseases such as innate immune deficiency syndrome. Furthermore, the present invention also provides a technique for efficiently conducting gene therapy.

### BRIEF DESCRIPTION OF THE DRAWINGS

[Fig. 1]

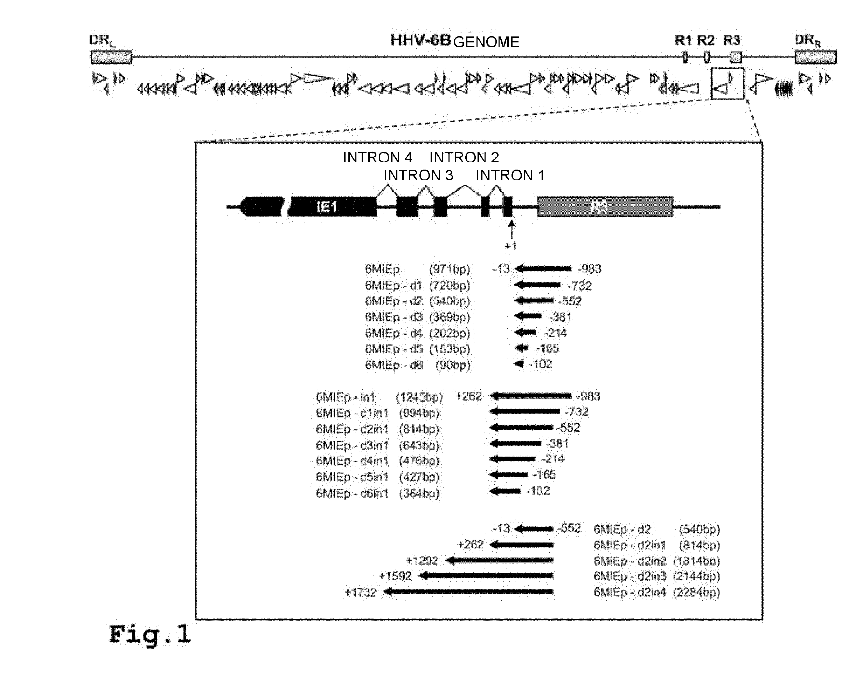

   Fig. 1 depicts a genetic map of HHV-6B genome. Triangles represent open reading frame regions. An enlarged view of around an IE1 region is illustrated, where regions of an MIE promoter and MIE promoter variants, which are amplified by PCR, are represented by arrows, respectively. Upper rows represent the MIE promoter and 5'-end-deleted variants, respectively. Middle rows represent promoter variants obtained by extending them to intron 1, respectively. Lower rows represent promoter variants obtained by extending the 5'-end 431-bp-deleted MIE promoter (6MIEp-d2) from intron 1 to intron 4, respectively. Numerals on the arrows represent relative positions: negative numerals for upstream positions and positive values for downstream positions with respect to the transcription initiation point of IE1 at +1. Positions of the respective introns are as follows: Intron 1: +63 to +220, Intron 2: +361 to +1163, Intron 3: +1297 to +1383, and Intron 4: +1595 to +1705.
[Fig. 2]

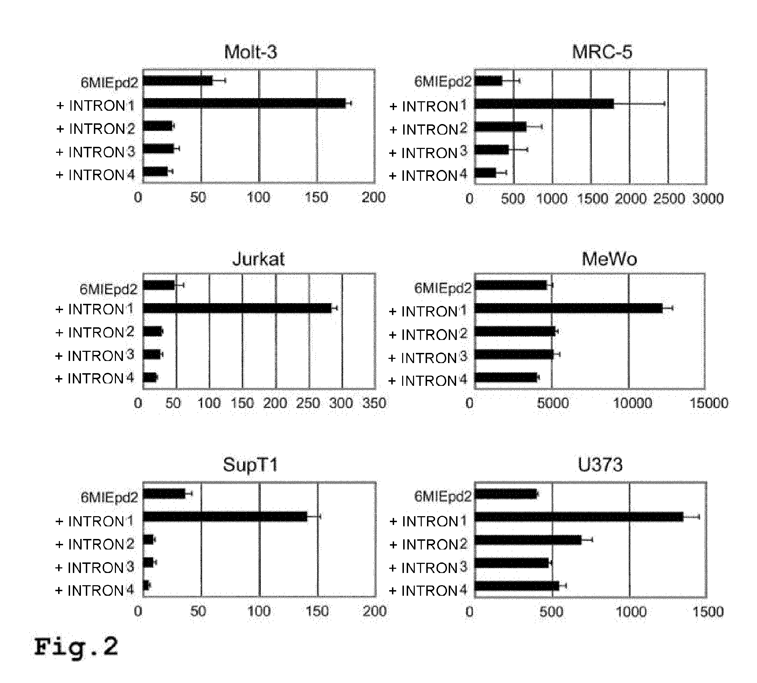

   Fig. 2 depicts a comparison of promoter activities of MIE promoter variants extended from intron 1 to intron 4 of the IE1. Each MIE promoter variant was cloned into pGL3 plasmid to prepare a reporter plasmid. The reporter plasmid was transfected into various cells, and the cells were then collected after 24 hours. Then, luciferase luminescence of the cells was measured, and the resulting relative values were represented in graph form. The cultured cells used were described on the upper sides of the respective graphs (from the upper left, MRC-5, MeWo, and U373; and from the lower left, Molt-3, Jurkat, and SupT1).
[Fig. 3]

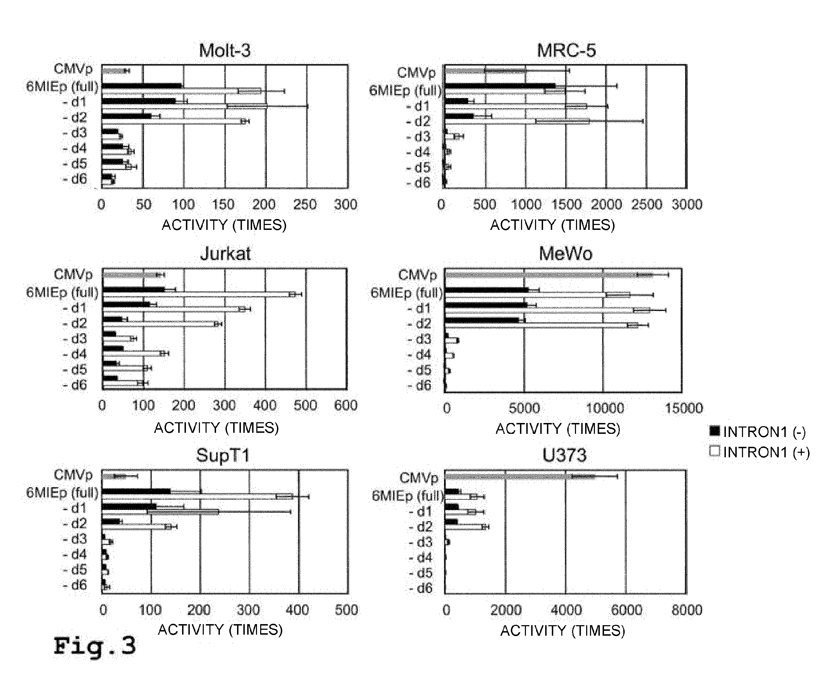

   Fig. 3 depicts a comparison of promoter activities of an MIE promoter and 5'-end-deleted MIE promoter variants in the presence or absence of intron 1. A CMV promoter and each of MIE promoter variants were cloned into pGL3 plasmid to prepare a reporter plasmid. The reporter plasmid was transfected into various cells, and the cells were then collected after 24 hours. Then, luciferase luminescence of the cells was measured, and the resulting relative values were represented in graph form. The cultured cells used were described on the upper sides of the respective graphs (from the upper left, MRC-5, MeWo, and U373; and from the lower left, Molt-3, Jurkat, and SupT1) .
[Fig. 4]

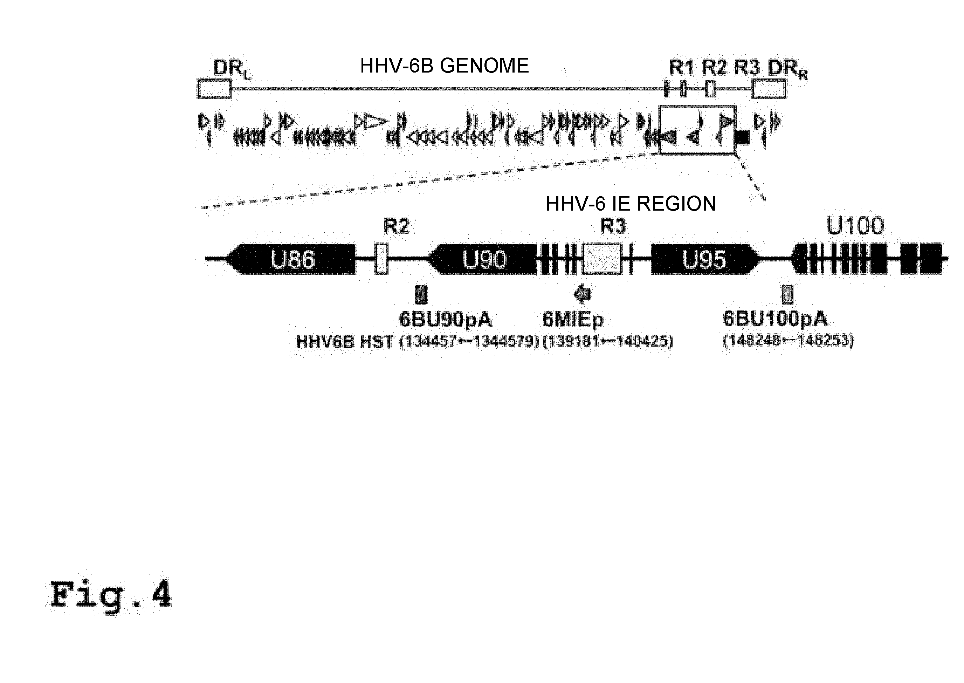

   Fig. 4 depicts a genetic map of HHV-6B genome. An enlarged view of an IE region is illustrated. Regions of an MIE promoter (including the first intron), U90 poly-A addition sequence, and U95 poly-A addition sequence are illustrated. Numerals in parentheses represent base numbers of the respective regions in HHV-6B strain HST <base numbers in the DNA sequence of Genbank accession No. AB021506 are represented>.
[Fig. 5]

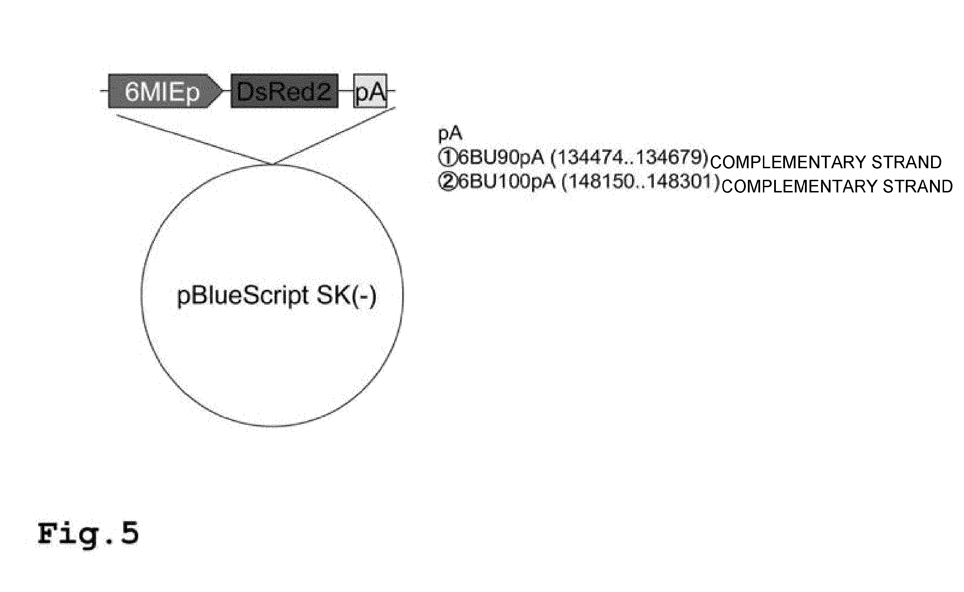

   Fig. 5 depicts a schematic diagram of a DsRed expression cassette including an MIE promoter. An expression cassette was produced by linking a gene encoding red fluorescence protein DsRed2 to the downstream side of the MIE promoter (including the first intron), and linking a region containing U90 or U100 poly-A addition sequence to the further downstream side thereof, followed by being inserted into pBlueScript SK (-) plasmid. Numerals in parentheses represent base numbers of the respective regions in HHV-6B strain HST, where each region actually contains the poly-A sequence inserted into the expression cassette <base numbers in the DNA sequence of Genbank accession No. AB021506>.

### MODE FOR CARRYING OUT THE INVENTION

Hereinafter, preferred embodiments of the present invention will be described. It should be understood that expression of a singular form also includes concept of a plural form thereof unless otherwise mentioned, throughout the present specification. Therefore, it should be understood that an article in a singular form (e.g., "a", "an", "the" etc. in the case of English) also includes concept of a plural form thereof unless otherwise mentioned. In addition, it should be understood that the terms used in the present specification are used in the meaning usually used in the art unless otherwise mentioned. Therefore, unless defined elsewhere, all the terminology, and the scientific and technical terms used in the present specification have the same meanings as those that are generally understood by those skilled in the art to which the present invention pertains. In the case of contradiction, the present specification (including definitions) prevails.

### (Definition)

The definitions of terms particularly used in the present specification are described below.

As used in the present specification, "HHV" refers to a human herpes virus, of which there are types 1, 2, 3, 4, 5, 6, 7, 8 and the like.

As used in the present specification, the term "herpesvirus" includes all of HHV-6A, HHV-6B, and HHV-7, and both their wild-types and recombinant types unless otherwise mentioned. As used in the present specification, the term "HHV-6 (human herpes virus-6)" includes HHV-6A and HHV-6B, and both their wild-types and recombinant types unless otherwise mentioned. HHV6 belongs to the same subgenus β as that of cytomegalovirus HHV-5, and HHV-6B is a causative virus of exanthema subitum. It is said that approximately all Japanese will have been infected therewith by the age of two years old.

The terms "protein", "polypeptide", "oligopeptide", and "peptide" used in the present specification are used in the same meaning in this specification, and each refers to a polymer of amino acids in arbitrary length.

The term "polynucleotide", "oligonucleotide", and "nucleic acid" used in the present specification are used in the same meaning in this specification, and each refers to a polymer of nucleotides in arbitrary length. Unless otherwise indicated, a particular nucleic acid sequence also implicitly encompasses conservatively-modified variants thereof (e.g., degenerate codon substitutions) and complementary sequences as well as the sequence explicitly indicated. Specifically, degenerate codon substitutions may be produced by generating sequences in which the third position of one or more selected (or all) codons is substituted with mixed-base and/or deoxyinosine residues (Batzer et al., Nucleic Acid Res. 19:5081(1991); Ohtsuka et al., J. Biol. Chem. 260:2605-2608 (1985); Rossolini et al., Mol. Cell. Probes 8:91-98(1994)).

In the present specification, "gene" refers to a factor that defines a genetic trait. A gene is typically arranged in a given sequence on a chromosome. A gene that defines the primary structure of a protein is called a structural gene. A gene that regulates the expression of a structural gene is called a regulatory element. In the present specification, "gene" may refer to "polynucleotide", "oligonucleotide", and "nucleic acid" and/or "protein", "polypeptide", "oligopeptide" and "peptide". In the present specification, "open reading frame" or "ORF" in relation to a gene, refers to a reading frame which is one of three frames obtained by sectioning the base sequence of a gene at intervals of three bases, and has a start codon and a certain length without appearance of a stop codon, and has the possibility of actually coding a protein. The entire base sequence of the genome of herpesvirus has been determined, identifying at least 101 genes. Each of the genes is known to have an open reading frame (ORF).

In the present specification, "RNAi" is an abbreviation of RNA interference and refers to a phenomenon where a factor for causing RNAi, such as double-stranded RNA (also called dsRNA), is introduced into cells and mRNA homologous thereto is specifically degraded, so that the synthesis of gene products is suppressed, and techniques using the phenomenon. In the present specification, RNAi may have the same meaning as that of a factor which causes RNAi.

In the present specification, "a factor causing RNAi" refers to any factor capable of causing RNAi. In the present specification, "a factor causing RNAi to a gene" indicates that the factor causes RNAi relating to the gene and that the effect of RNAi is successfully achieved (e.g., suppression of expression of the gene). Examples of such a factor causing RNAi include, but are not limited to, RNA containing a double-stranded portion having a length of at least 10 nucleotides or variants thereof, which have a sequence having at least about 70*%* homology with a portion of the nucleic acid sequence of a target gene or a sequence hybridizable thereto under stringent conditions. Here, this factor may be preferably DNA containing a 3' protruding end, where more preferably the 3' protruding end has a length of 2 or more nucleotides (e.g., 2 to 4 nucleotides in length).

In the present specification, "corresponding" amino acid and nucleic acid refer to an amino acid and a nucleic acid, respectively, in a given polypeptide and nucleic acid molecule, which have, or are anticipated to have, a function similar to that of a predetermined amino acid and nucleic acid in a polypeptide and nucleic acid molecule as a reference for comparison. For example, in the case of ubiquitin, the corresponding amino acid and nucleic acid refer to an amino acid contributing in a similar manner to the catalytic activity and present in a similar location as in the sequence (for example, glycine at the C-terminus) which is responsible for linking lysine, and a nucleic acid encoding the same. For example, in the case of a nucleic acid sequence, the corresponding amino acid and nucleic acid may be the nucleic acid sequence or a similar portion which exerts a similar function to the particular portion which it encodes.

In the present specification, "corresponding" gene, promoter, and intron refer to a gene in a given species, which have, or are anticipated to have, a function similar to that of a predetermined gene in a species as a reference for comparison. When there are a plurality of genes having such a function, it refers to a gene having the same evolutionary origin. Therefore, a gene corresponding to a given gene may be an ortholog of the given gene. Therefore, genes corresponding to those such as herpes virus type 6B and tumor antigen, can be found in other organisms (for example, herpes virus type 7). Such a corresponding gene can be identified by techniques well-known in the art. Therefore, for example, a corresponding gene in a given animal can be found by searching a sequence database of the animal (e.g., herpes virus 6B) using the sequence of a reference gene (e.g., MIE promoter sequence, intron sequence, or the like of herpes virus 6A) as a query sequence. Alternatively, wet experiments can be used for screening a library to find out the same.

In the present specification, "isolated" substance (e.g., a biological factor, such as a nucleic acid or a protein) refers to a substance substantially isolated or purified from other substances (preferably, biological factors) in the environment in which such a substance is naturally-occurring (e.g., in the cells of an organism) (for example, the "isolated" substance means that, in the case of the nucleic acid, factors other than nucleic acids and nucleic acids containing nucleic acid sequences other than that of the nucleic acid of interest; and, in the case of the protein, factors other than proteins and proteins containing amino acid sequences other than that of the protein of interest). The term "isolated" nucleic acid and protein encompasses nucleic acids and proteins purified by standard purification techniques. Therefore, the isolated nucleic acid and protein encompasses chemically synthesized nucleic acids and proteins.

In the present specification, "purified" substance (e.g., a biological factor such as a nucleic acid or a protein) refers to one from which at least a portion of naturally accompanying factors has been removed. Therefore, ordinarily, the purity of a purified substance is higher than that of a substance in a normal state (i.e., concentrated).

In the present specification, "purified" and "isolated" mean that the same type of a substance is present preferably at least 75% by weight, more preferably at least 85% by weight, even more preferably at least 95% by weight, and most preferably at least 98% by weight.

In the present specification, "homology" in relation to a gene refers to the proportion of identity between two or more gene sequences. Therefore, the greater the homology between two given genes, the greater the identity or similarity between their sequences. Whether or not two genes have homology is determined by comparing their sequences directly or by a hybridization method under stringent conditions in the case of nucleic acid. When two gene sequences are directly compared with each other, these genes have homology if the DNA sequences of the genes have representatively at least 50% identity, preferably at least 70% identity, more preferably at least 80%, 90%*,* 95%, 96%, 97%, 98%, or 99% identity with each other.

In the present specification, "stringent hybridization conditions" refers to conditions commonly used and well-known in the art. By conducting a colony hybridization method, a plaque hybridization method, a Southern blot hybridization method, or the like using a polynucleotide selected from the polynucleotides of the present invention as a probe, such a polynucleotide can be obtained. Specifically, a polynucleotide to be hybridized under stringent conditions refers to a polynucleotide that can be identified by performing hybridization at 65°C in the presence of 0.7 to 1.0 M NaCI using a filter on which DNA derived from a colony or plaque is immobilized, and washing the filter at 65°C with a 0.1 to 2-fold concentration SSC (saline-sodium citrate) solution (1-fold concentration SSC solution is composed of 150 mM sodium chloride and 15 mM sodium citrate). Hybridization can be conducted in accordance with a method described in an experiment book, for example, Molecular Cloning 2nd ed., Current Protocols in Molecular Biology, Supplement 1-38, DNA Cloning 1: Core Techniques, A Practical Approach, Second Edition, Oxford University Press (1995). Here, sequences hybridizing under stringent conditions exclude, preferably, sequences containing only A or T. "Hybridizable polynucleotide" refers to a polynucleotide that can hybridize to other polynucleotides under the above-described hybridization conditions. Specific examples of the hybridizable polynucleotide include a polynucleotide having a homology of at least 60% to the base sequence of DNA encoding a polypeptide having an amino acid sequence specifically disclosed in the present specification, preferably a polynucleotide having a homology of at least 80%, and more preferably a polynucleotide having a homology of at least 95%.

In the present specification, the identity comparison and homology calculation of base sequences are performed using a sequence-analyzing tool BLAST with the default parameters. An identity search may be conducted, for example, using NCBI BLAST 2.2.9 (published on May 12, 2004). The value of identity in the present specification usually refers to a value as a result of alignment with the BLAST as described above using the default parameters. However, if the change of parameters results in higher values, then the highest value is employed as the value of identity. When a plurality of regions is evaluated for identity, the highest value is employed as the value of the identity.

In the present specification, "search" indicates that a given nucleic acid base sequence is utilized to find other nucleic acid base sequences having a specific function and/or property either electronically or biologically, or using other methods. Examples of the electronic search include, but are not limited to, BLAST (Altschul et al., J. Mol. Biol. 215:403-410 (1990)), FASTA (Pearson & Lipman, Proc. Natl. Acad. Sci., USA 85:2444-2448 (1988)), Smith and Waterman method (Smith and Waterman, J. Mol. Biol. 147:195-197 (1981)), and Needleman and Wunsch method (Needleman and Wunsch, J. Mol. Biol. 48:443-453 (1970)), and the like. Examples of the biological search include, but are not limited to, stringent hybridization, a microarray in which genomic DNA is attached to a nylon membrane or the like or a microarray (microarray assay) in which genomic DNA is attached to a glass plate, PCR and in-situ hybridization. In the present specification, it is intended that promoters used in the present invention encompass a sequence corresponding to those identified by such an electronic or biological search.

In the present specification, "expression" of a gene, a polynucleotide, a polypeptide, or the like, indicates that the gene or the like is affected by a predetermined action in vivo to be changed into another form. Preferably, it indicates that genes, polynucleotides, or the like are transcribed and translated into polypeptides. In one aspect of the expression, genes may be transcribed into mRNA. More preferably, these polypeptides may have post-translational processing modifications.

In the present specification, amino acids may be referred to with the generally known three-letter abbreviation or the one letter-abbreviation proposed by the IUPAC-IUB Biochemical Nomenclature Commission. Nucleotides may also be referred to with the generally known one-letter abbreviations which are generally accepted.

In the present specification, "fragment" refers to a polypeptide or polynucleotide having a sequence length ranging from 1 to n-1 with respect to the full length of a polypeptide or polynucleotide (having a length of n). The length of the fragment can be appropriately changed depending on the purpose. For example, in the case of polypeptides, the lower limit of the length of the fragment includes 3, 4, 5, 6, 7, 8, 9, 10, 15, 20, 25, 30, 40, 50 or more amino acids. Lengths represented by integers which are not herein specified (e.g., 11) may also be appropriate as a lower limit. For example, in the case of polynucleotides, the lower limit of the length of the fragment includes 5, 6, 7, 8, 9, 10, 15, 20, 25, 30, 40, 50, 75, 100, 200, 300, 400, 500, 600, 600, 700, 800, 900, 1000 or more nucleotides. Lengths represented by integers which are not herein specified (e.g., 11) may also be appropriate as a lower limit.

The polypeptide used in the present invention may have at least one (for example, one or several) amino acid substitutions, additions and/or deletions in the amino acid sequence, as long as it has a substantially identical function as a naturally-occurring polypeptide.

In the present specification, "variant" refers to a substance, such as a polypeptide, polynucleotide, or the like, which differs partially from the original substance. Examples of such a variant include a substitution variant, an addition variant, a deletion variant, a truncated variant, and an allelic variant. The term "allele" refers to a genetic variant located at a locus identical to a corresponding gene, where the two genes are distinguishable from each other. Therefore, the term "allelic variant" refers to a variant which has an allelic relationship with a given gene. The term "species homolog or homolog" refers to one that has an amino acid or nucleotide homology with a given gene in a given species (preferably at least 60% homology, more preferably at least 80%, at least 85%, at least 90%, and at least 95% homology). A method for obtaining such a species homolog is clearly understood from the description of the present specification. The term "orthologs" (also called orthologous genes) refers to genes in different species derived from a common ancestry (due to speciation). For example, in the case of a hemoglobin gene family having a multigene structure, human and mouse-oc hemoglobin genes are orthologs, while the human-α hemoglobin gene and the human-β hemoglobin gene are paralogs (genes arising from gene duplication). Orthologs are useful for estimation of molecular phylogenetic trees. Therefore, the orthologs of the present invention may be useful in the present invention.

In the present specification, "functional variant" refers to a variant which retains a biological actiVity (in particular, promoter activity) which the sequence of standard is responsible for.

In the present specification, "conservative (or conservatively modified) variant" applies to both amino acid and nucleic acid sequences. With respect to particular nucleic acid sequences, conservatively modified variants refer to nucleic acids that encode identical or essentially identical amino acid sequences, and refer to essentially identical sequences if the nucleic acids do not encode amino acid sequences. Because of the degeneracy of the genetic code, a large number of functionally identical nucleic acids encode any given protein. For example, codons GCA, GCC, GCG and GCU all encode amino acid alanine. Thus, at every position where alanine is specified by a codon, the codon can be altered to any of the corresponding codons described without altering the encoded polypeptide. Such nucleic acid variations are "silent alterations (variations)" which represent one species of conservatively modified variation. In a nucleic acid, for example, a conservative substitution can be confirmed by measuring a promoter activity.

In the present specification, in order to prepare genes that encode functionally equivalent polypeptides, amino acid additions, deletions, or modifications can be performed in addition to amino acid substitutions. Amino acid substitution(s) refers to the replacement of at least one amino acid of an original peptide with different amino acids, such as the replacement of 1 to 10 amino acids, preferably 1 to 5 amino acids, and more preferably 1 to 3 amino acids of an original peptide with different amino acids. Amino acid addition (s) refers to the addition of at least one amino acid to an original peptide chain, such as the addition of 1 to 10 amino acids, preferably 1 to 5 amino acids, and more preferably 1 to 3 amino acids to an original peptide chain. Amino acid deletion(s) refers to the deletion of at least one amino acid from an original peptide, such as the deletion of 1 to 10 amino acids, preferably 1 to 5 amino acids, and more preferably 1 to 3 amino acids from an original peptide. Examples of amino acid modification include, but are not limited to, amidation, carboxylation, sulfation, halogenation, alkylation, glycosylation, phosphorylation, hydroxylation, and acylation (e.g., acetylation). Amino acids to be substituted or added may be naturally-occurring or nonnaturally-occurring amino acids, or amino acid analogs. Naturally-occurring amino acids are preferred.

A nucleic acid form of the polypeptide to be expressed in the present specification refers to a nucleic acid molecule that allows expression of a protein form of the polypeptide. Such a nucleic acid molecule may be one in which a part of the sequence of the nucleic acid is deleted or is substituted with other base(s), or may be one in which a part of other nucleic acid sequences is inserted, as described above, as long as a polypeptide to be expressed has substantially the same activity as that of the naturally-occurring polypeptide. Alternatively, other nucleic acids may be linked to the 5' end and/or 3' end of the nucleic acid. The nucleic acid molecule may be one that is hybridizable to a gene encoding a polypeptide under stringent conditions and encodes a polypeptide having substantially the same function as that of the polypeptide. Such a gene is known in the art and can be used in the present invention.

Such a nucleic acid can be obtained by the well-known PCR method, and can also be chemically synthesized. This method may be combined with, for example, a site-specific mutagenesis method, a hybridization method, or the like.

In the present specification, "substitution, addition or deletion" for a polypeptide or a polynucleotide refers to the substitution, addition or deletion of an amino acid or its substitute, or a nucleotide or its substitute with respect to the original polypeptide or polynucleotide. Techniques for such substitution, addition, or deletion are well-known in the art, and examples of the technique include site-specific mutagenesis techniques. The number of substitutions, additions, or deletions may be arbitrarily determined as long as it is one or more. The number can be made large as long as a variant having such substitutions, additions or deletions can maintain an intended function. For example, such a number may be one or several, and preferably within 20% or 10% of the full length sequence, or not more than 100, not more than 50, not more than 25, or the like.

### (Promoter)

In the present specification, "promoter (or promoter sequence)" refers a DNA region that determines the initiation site of transcription of a gene and directly regulates the frequency of transcription, and is a base sequence to which RNA polymerase usually binds to initiate transcription. Accordingly, in the present specification, a portion having the function of a promoter of a gene refers to "a promoter portion". A promoter region can be deduced by predicting the protein coding region in a genomic base sequence using DNA analysis software. Deduced promoter regions are usually located at the upstream of a structural gene although it varies depending on the structural gene, and is not limited thereto, and may also be the downstream of the structural gene.

In the present specification, "MIE" (gene) refers to a major immediate early gene, which is a gene that is immediately transcribed by a transcription factor derived from a host or a virion after viral infection.

In the present specification, "MIE promoter" or "MIEp" refers to a major immediate early promoter, which is a promoter of a gene that is immediately transcribed by a transcription factor derived from a host or a virion after viral infection. The MIE gene may be identified by RT-PCR using RNA extracted from an infected cell treated with cycloheximide (CHX).

In the present specification, "6MIE" (gene) refers to a major immediate early gene of human herpes virus 6 (HHV-6) (particularly, HHV-6B). In the present specification, "IE1 of 6MIE" refers to a gene that encodes an IE1 protein among proteins encoded by the major immediate early gene of HHV-6. The transcription initiation point of IE1 (SEQ ID NO. 41) of 6MIE is illustrated as +1 in Fig.1.

In the present specification, "6MIE promoter" or "6MIEp" refers to a promoter of a major immediate early gene (MIE) of human herpes virus 6 (particularly HHV-6).

In the present specification, the identification method of a promoter is as follows: that is, some sequences in the vicinity of the structural gene are screened (for example, using an expression cassette described in the examples), and the sequence having a gene expression promoting activity is mapped. Consequently, a sequence having a significant promoting activity may be identified. Usually, it is located at the upstream of the structural gene in many cases, but is not limited thereto.

In the present specification, "MIE promoter of HHV-6B" refers to any sequence having a promoter activity in SEQ ID NO: 1. Preferably, the promoter has position -770 to position -1 from the transcription initiation point in SEQ ID NO: 1. Examples of the sequence include, but are not limited to, SEQ ID NO: 1 or a sequence corresponding thereto. In the expression control of HHV-6B gene, it is preferable to be located in the region at -530 to -383 from the upstream, and preferably in the region of -1007 to -383 from the transcription initiation point, and the base sequence thereof includes sequences set forth in SEQ ID NOs: 5, 6, and the like. Amongst them, it has been elucidated that NF-KB and AP-1 motifs (-541 to -534 from the transcription initiation point as the origin, corresponds to NF-κB motif sequence, and -426 to -416 and -196 to -186 correspond to the AP-1 motif sequence) may be motifs from experiments of base sequence substitution. Therefore, preferably, the MIE promoter of HHV-6B of the present invention includes: (a) a polynucleotide having the base sequence set forth in SEQ ID NO: 1, or a base sequence corresponding thereto or a fragment sequence thereof; (b) a polynucleotide of an allelic variant of the base sequence set forth in SEQ ID NO: 1 or a base sequence corresponding thereto or a fragment sequence thereof; (c) a polynucleotide which hybridizes the polynucleotide of any of (a) and (b) under stringent conditions and has a biological activity; or (d) a polynucleotide of any of (a) to (c) or a polynucleotide which contains the base sequence with at least 70% identity to a complement sequence thereof, and has a biological activity.

In the present specification, "constructive" expression of a promoter refers to a trait in which expression is found at a substantially predetermined amount in any tissue of an organism during any stage in the course of the development of the organism. Specifically, when northern blot analysis is carried out under conditions similar to those in the examples described in the present specification, if substantially the same expression is observed in the same or corresponding site thereof on any time points (e.g., two or more time points such as day 5 and day 15), the expression is regarded as being constructive by the definition in the present invention. Constructive promoters are believed to play a role in the homeostasis of organisms in a normal growth environment. These traits can be determined by extracting RNA from an arbitrary portion of an organism and subjecting the RNA to northern blot analysis to analyze expression amounts or subjecting the expressed protein to western blot to determine the quantity of the protein.

In the present specification, the "enhancer" or "promoter of enhancer" may be used so as to enhance the expression efficiency of a gene of interest. A plurality of enhancers or a single enhancer may be used, or no enhancer may be used. A region in an intron or promoter which enhances the activity of the promoter may also be referred to as an enhancer as long as it has the activity of enhancing the expression efficiency of a gene.

In the present specification, "intron" is a gene region provided as an intervening sequence that is present in DNA and included in a primary transcript, but not included in the final functional mature RNA, and removed by splicing.

In the present specification, "operatively linked" or "operative link" refers to the fact that the expression (operation) of a desired sequence is located under the control of a transcription regulation sequence (e.g., promoter or enhancer) or a translation regulation sequence. In order that a promoter is operatively linked to a gene, the promoter is usually located immediately at the upstream of the gene, but is not necessarily located in a flanking manner.

### (Nucleic acid construct)

In the present specification, "nucleic acid construct" and "gene cassette" are interchangeably used to refer to a nucleic acid sequence including: a nucleic acid molecule(for example, DNA, RNA) encoding a gene; and a control sequence (e.g., a promoter) operatively linked thereto (such that it can control expression of the nucleic acid) according to necessity, and also refer to a nucleic acid molecule including: a control sequence (e.g., a promoter); and a heterologous gene operatively linked thereto (i.e., in frame) according to necessity. It is intended that the use of this cassette or the construct optionally in combination with other regulatory elements is encompassed in the present invention. Preferable expression cassettes or nucleic acid constructs are those cleaved by a specific restriction enzyme and are easy for recovery.

When a gene is mentioned in the present specification, "vector." refers to one capable of transferring a polynucleotide sequence of interest into a target cell. Examples of such a vector include those capable of self-replication in a host cell or incorporation into a chromosome, and containing a promoter at a site suitable for transcription of the polynucleotide of the present invention, such as a prokaryotic cell, yeast, an animal cell, a plant cell, an insect cell, an individual animal, and an individual plant. In the present specification, for example, BAC vectors may be used. The BAC vector refers to a plasmid produced based on the F plasmid of E. coli, and is capable of propagating and stably maintaining a DNA fragment of about 300kb or greater in size, in a bacteria such as E.coli or the like. The BAC vector includes at least a region essential for replication of BAC vectors. Examples of such a region essential for replication include oriS, a replication initiation point of F plasmid, or a variant thereof.

As used in the present specification, "selective marker" refers to a gene that functions as an indicator for selecting a host cell including a nucleic acid construct or a vector. Examples of the selective markers include, but are not limited to, fluorescent markers, luminescent markers and drug selective markers. Examples of the "fluorescent markers" include, but are not limited to, genes encoding fluorescence proteins such as green fluorescent protein (GFP), cyan fluorescent protein (CFP), yellow fluorescent protein (YFP), and red fluorescent protein (dsRed). Examples of the "luminescent markers" include, but are not limited to, genes encoding luminescent proteins such as luciferases. Examples of the "drug selective markers" include, but are not limited to, hypoxanthine guanine phosphoribosyl transferase (hprt), dihydrofolate reductase gene, glutamine synthase gene, aspartate transaminase, metallothionein (MT), adenosine aminase (ADA), AMP deaminase (AMPD1, 2), xanthine-guanine-phosphoribosyl transferase, UMP synthase, P-glycoprotein, asparagine synthase, and ornithine decarboxylase. Examples of a combination of these drug selective markers and a drug to be used include, but not limited thereto, genes that encode proteins, such as a combination of dihydrofolate reductase (DHFR) gene and methotrexate (MTX); a combination of glutamine synthase (GS) gene and methionine sulfoximine (Msx); a combination of aspartate transaminase (AST) gene and N-phosphonacetyl-L-aspartate (PALA); a combination of MT gene and cadmium (Cd²⁺); a combination of adenosine deaminase (ADA) gene and adenosine, alanosine, 2'-deoxycoformycin; a combination of AMP deaminase (AMPD1, 2) gene and adenine, azaserine and coformycin; a combination of xanthine-guanine-phosphoribosyl transferase gene and mycophenolic acid; a combination of UMP synthase gene and 6-azauridine, pyrazofuran; a combination of P-glycoprotein (P-gp, MDR) gene and multi drugs; a combination of aspartate synthase (AS) gene and β-aspartyl hydroxamic acid or albizziin; ornithine decarboxylase (ODC) gene and α-difluoromethyl-ornithine (DFMO).

As used in the present specification, "expression vector" refers to a nucleic acid sequence including a structural gene and a promoter for regulating expression thereof, and in addition, various regulatory elements in a state that allows them to operate within host cells. The regulatory element may include, preferably, terminators, selective markers such as drug-resistance genes (e.g., kanamycin resistant gene and hygromycin resistant gene), and enhancers. It is well-known in the art that a type of expression vector of an organism such as an animal and a kind of a regulatory element to be used may vary depending on the host cells. As used in the present specification, "recombinant vector" refers to a vector capable of transferring a polynucleotide sequence of interest to a target cell. Examples of such a vector include those capable of self-replication in a host cell or incorporation into a chromosome, and containing a promoter at a site suitable for transcription of the polynucleotide of the present invention, such as a prokaryotic cell, yeast, an animal cell, a plant cell, an insect cell, an individual animal, and an individual plant.

In the present specification, "terminator" refers to a sequence which is located at the downstream of a protein-encoding region of a gene and which is involved in the termination of transcription when DNA is transcribed into mRNA, and the addition of a poly-A sequence. It is known that a terminator contributes to the stability of mRNA, and has an influence on the amount of gene expression. Examples of the terminator include, but are not limited to, a sequence including AATAAA.

In the present specification, "foreign gene" refers to, in a given organism, a gene which does not naturally occur in the given organism. Such a foreign gene may be a gene modified from a gene which naturally occurs in the given organism, or a gene which naturally occurs in an organism that is different from the given organism (e.g., ADA gene), or an artificially synthesized gene, or a complex thereof (e.g., a fusion). An organism containing such a foreign gene may express a genetic product which is not expressed in nature. For example, a recessive gene to be deleted (for example, ADA gene, PNP gene, γc chain gene, TAP gene, MHC II gene, X-linked WASP, CD40 ligand, PI3K-like gene, DNA helicase) may be used as a foreign gene.

In the present specification, the foreign gene may be of cytokine. As used in the present specification, "cytokine" is defined as in the broadest sense used in the art, and a physiologically active substance which is produced from a cell and acts on the same cell or a different cell. Cytokines are generally a protein or a polypeptide, and have a controlling action of immunological response, regulation of endocrine system, regulation of the nerve system, antitumor activity, antiviral activity, regulatory action of cell proliferation, regulatory action of cellular differentiation and the like. In the present specification, cytokines may exist in a protein form or nucleic acid form, or in any other forms, and at the actual time of action, cytokines usually mean a protein form. As used in the present specification, "growth factor" refers to a substance which promotes or controls the growth of a cell. Growth factors are also called as growth- or development-stimulating substances. Growth factors may substitute the action of serum macromolecular substances by addition to a medium in a cell culture or a tissue culture. Many growth factors have been found to function as a control factor of a differentiation state other than growth of a cell. The cytokines typically include interleukins, chemokines, hematopoietic factors such as colony stimulation factors, tumor necrosis factors, and interferons. Examples of the growth factors typically include platelet derived growth factor (PDGF), epidermal growth factor (EGF), fibroblast growth factor (FGF), hepatocytic growth factor (HGF), and vessel endothelial growth factor (VEGF), which show growth activity.

In the present invention, those having homology with the foreign gene of a native form as described above may be used as a foreign gene to be expressed. Examples of the foreign gene with such homology include, but are not limited to, nucleic acid molecules having nucleic acid sequences with an identity or similarity of at least about 30*%*, at least about 35%, at least about 40%, at least about 45%, at least about 50%, at least about 55%*,* at least about 60%, at least about 65%, at least about 70%, at least about 75%, at least about 80%, at least about 85%, at least about 90%, at least about 95%, at least about 99%, or polypeptide molecules having amino acid sequence of an identity or similarity of at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, to a comparative foreign gene when conducting comparison using default parameters of Blast.

In the present specification, "expression" of a gene product, such as a gene, a polynucleotide, a polypeptide, or the like, indicates that the gene or the like is affected by a predetermined action in vivo to be changed into another form. Preferably, it indicates that genes, polynucleotides, or the like are transcribed and translated into polypeptides. In one aspect of the expression, genes may be transcribed into mRNA. More preferably, these polypeptides may have post-translational processing modifications.

Accordingly, in the present specification, "reduction" of "expression" of a gene, a polynucleotide, a polypeptide or the like refers to when the factor of the present invention is allowed to act, the amount of expression is significantly reduced compared to that when the factor is not allowed to act. Preferably, the reduction of expression includes a reduction in an amount of polypeptide expression. In the present specification, "increase" of "expression" of a gene, a polynucleotide, a polypeptide or the like refers to when the factor of the present invention is allowed to act, the amount of expression is significantly increased compared to when the factor is not allowed to act. Preferably, the increase of expression includes an increase in an amount of polypeptide expression. In the present specification, "induction" of "expression" of a gene refers to an increase in an amount of expression of the gene by acting a factor on a cell. Accordingly, the induction of expression encompasses that the expression of the gene is observed when the expression of the gene is not observed at all, and that the expression of the gene is increased when the expression of the gene has already been observed.

In the present specification, "specific expression" of a gene refers to expression in a different level (preferably in a higher level) in a specific site or period of time in a plant than that of the other site or period of time. The term specific expression may refer to expression in a certain site (specific site) or may also refer to expression other than the certain site. Preferably, the term specific expression refers to expression in the certain site only.

As used in the present specification, any of methods for introducing a recombinant vector can be used as long as they are methods for introduction of DNA, and examples thereof include a calcium chloride method, an electroporation method [Methods. Enzymol., 194, 182 (1990)], a lipofection method, a spheroplast method [Proc. Natl. Acad. Sci. USA, 84, 1929 (1978)], a lithium acetate method [J. Bacteriol., 153, 163 (1983)], and a method described in Proc. Natl. Acad. Sci. USA, 75, 1929 (1978) .

Transitional expression of Cre enzyme, DNA mapping on the chromosomes, and the like, used in a method for removing a genome, genomic locus or the like used in the present specification are well-known in the art as described in "FISH Experimental Protocol: from human/genomic analysis to chromosomal/genetic diagnosis" in Cell Engineering, special issue, Experimental Protocol Series, supervised by Kenichi Matsubara, Hiroshi Yoshikawa, SHUJUNSHA Co., Ltd. (Tokyo), and the like.

In the present specification, "detection" or "quantification" of gene expression (e.g., mRNA expression, polypeptide expression) may be attained by an appropriate method, including a mRNA measurement method and an immunological measurement method. Examples of molecular biological measurement methods include Northern blotting methods, dot blotting methods, and PCR methods. Examples of the immunological measurement methods include ELISA methods where a microtiter plate may be used, RIA methods, fluorescent antibody methods, Western blotting methods, and immunohistological staining methods. Examples of quantification methods include ELISA methods, and RIA methods. A gene analysis method using an array (e.g., a DNA array, a protein array) may also be used. The DNA array is widely reviewed in Cell Engineering, special issue, "DNA Microarray and Up-to-date PCR Method", edited by SHUJUNSHA Co., Ltd. The protein array is described in detail in Nat Genet. 2002 Dec; 32 Suppl:526-32. Examples of methods for analyzing gene expression include, but are not limited to, RT-PCR methods, RACE methods, SSCP methods, immunoprecipitation methods, two-hybrid systems, and in vitro translation methods in addition to the above-described techniques. Other analysis methods are described in, for example, "Genome Analysis Experimental Method, Yusuke Nakamura's Lab-Manual, edited by Yusuke Nakamura, YODOSHA Co., Ltd. (2002), and the like. All of the above descriptions are herein incorporated by reference.

In the present specification, "expression amount" refers to the amount of a polypeptide or mRNA expressed in a subject cell. Examples of such an expression amount include an amount of expression of the polypeptide of the present invention in a protein level evaluated by any appropriate method using the antibody of the present invention, including immunological measurement methods such as an ELISA method, an RIA method, a fluorescent antibody method, a Western blotting method, an immunohistological staining method, and the like, or an amount of expression of the polypeptide of the present invention in a mRNA level evaluated by any appropriate method, including molecular biological measurement methods such as a Northern blotting method, a dot blotting method, a PCR method, and the like. The term "change in amount of expression" indicates that an increase or decrease in an amount of expression of the polypeptide of the present invention in a protein level or in a mRNA level evaluated by any appropriate method including the above-described immunological measurement method or molecular biological measurement method.

As used in the present specification, the terms "transformation", "transduction", and "transfection" are used interchangeably unless otherwise mentioned, and refer to introduction of a nucleic acid into host cells. As a transformation method, any technique for introducing DNA into host cells can be used, and examples thereof include various well-known techniques such as an electroporation method, a method using particle gun (gene gun), and a calcium phosphate method.

The term "transformant" refers to the whole or a part of a living organism, such as a cell, which is produced by transformation. Examples of the transformant include prokaryotic cells, yeast, animal cells, plant cells, and insect cells. Transformants may be referred to as transformed cells, transformed tissue, transformed hosts, or the like, depending on the subject, and in the present specification, all of the forms are encompassed; however, a particular form may be specified in a particular context.

Examples of the prokaryotic cells include those of the genera Escherichia, Serratia, Bacillus, Brevibacterium, Corynebacterium, Microbacterium, and Pseudomonas, including Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No.49, Escherichia coli W3110, Escherichia coli NY49, Escherichia coli BL21(DE3), Escherichia coli BL21(DE3)pLysS, Escherichia coli HMS174(DE3), Escherichia coli HMS174(DE3)pLysS, Serratia ficaria, Serratia fonticola, Serratia liquefaciens, Serratia marcescens, Bacillus subtilis, Bacillus amyloliquefaciens, Brevibacterium ammmoniagenes, Brevibacterium immariophilum ATCC14068, Brevibacterium saccharolyticum ATCC14066, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC15354, Pseudomonas sp.D-0110, and the like.

Examples of the animal cells include cord blood mononuclear cells, peripheral blood mononuclear cells, and Sup-T1 cells.

In the present specification, "animal" is used in its broadest sense in the art and refers to vertebrates and invertebrates. Examples of the animals include, but are not limited to, the class Mammalia, the class Aves, the class Reptilia, the class Amphibia, the class Pisces, the class Insecta, and the class Vermes.

In the present specification, "tissue" in relation to organisms refers to an aggregate of cells having a certain similar function. Therefore, a tissue may be a portion of an organ (body organ). Organs (body organs) usually have cells having the same function in many cases, but may have coexisting cells having slightly different functions. Therefore, in the present specification, tissues may have various kinds of cells in a coexisting manner as long as a certain property is shared by the cells.

In the present specification, "body organ (organ)" refers to a structure which has a single independent form and in which one or more tissues are combined together to perform a specific function. In animals, examples thereof include, but are not limited to, stomach, liver, intestine, pancreas, lung, airway, nose, heart, artery, vein, lymph node (lymphatic system), thymus, ovary, eye, ear, tongue, and skin.

In the present specification, "transgenic" refers to incorporation of a specific gene into an organism or an organism incorporated such a gene (e.g., plants or animals (mice, etc.)). Among transgenic organisms, one with a deleted or suppressed gene refers to a knockout organism.

When the organisms of the present invention are animals, the transgenic organisms can be produced by using a production technique of a transgenic organism utilizing a microinjection method (a trace amount injection method), a virus vector method, an ES cell method (embryonic stem cell method), a sperm vector method, a chromosome fragment introducing method (transsomic method), an episome method, or the like. These production techniques of transgenic animal are well-known in the art.

As used in the present specification, "screening" refers to selection of a substance, a host cell, a virus, or the like having a given specific property of interest from a number of candidates using a specific operation/evaluation method. It will be understood that the present invention also encompasses viruses having a desired activity obtained by screening.

In the present specification, "chip" and "microchip" are used interchangeably to refer to a micro-integrated circuit that has versatile functions and constitutes a portion of a system. Examples of the chip include, but are not limited to, DNA chips, protein chips, and cell chips.

The herpesvirus promoters of the present invention can be used as an ingredient of a pharmaceutical composition for the treatment, prevention, and/or therapy of lymphatic or hemic diseases, immune diseases, and infectious diseases.

In the present specification, "effective amount" in relation to a drug refers to an amount which causes the drug to exhibit its intended efficacy. In the present specification, an effective amount corresponding to the smallest concentration may be referred to as a minimum effective amount, among such effective amounts. Such a minimum effective amount is well-known in the art. Typically, the minimum effective amount of a drug has been determined or can be determined as appropriate by those skilled in the art. The determination of such an effective amount can be achieved by the use of an animal model, or the like in addition to actual administration. The present invention is also useful for the determination of such an effective amount.

In the present specification, "pharmaceutically acceptable carrier" refers to a substance which is used for production of a pharmaceutical agent or an agricultural chemical (e.g., an animal drug), and has no adverse effect on effective ingredients. Examples of such a pharmaceutically acceptable carrier include, but are not limited to, antioxidants, preservatives, colorants, flavoring agents, diluents, emulsifiers, suspending agents, solvents, fillers, bulking agents, buffers, delivery vehicles, excipients, and/or agricultural or pharmaceutical adjuvants.

The kind and amount of a drug to be used in the treatment method of the present invention can be easily determined by those skilled in the art based on information obtained by the method of the present invention (e.g., information relating to a disease) in view of the purpose of use, the target disease (kind, severity, etc.), the patient's age, weight, sex, and past history, the form and kind of a site of a subject to be administered, or the like. The frequency of subjecting a subject (patient) to the monitoring method of the present invention can also be easily determined by those skilled in the art in view of the purpose of use, the target disease (kind, severity, etc.), the patient's age, size, weight, and past history, the progression of the therapy, and the like. Examples of the frequency of monitoring the state of a disease include once per day to once per several months (e.g., once per week to once per month). Preferably, monitoring is preferably performed once per week to once per month with reference to the progression.

In the present specification, "instruction(s)" " refers to a description of the treatment method of the present invention for a person who performs administration, such as a medical doctor and a patient. The instructions state administration of the pharmaceutical agent of the present invention, for example, immediately after or before radiation therapy (e.g., within 24 hours). The instructions are prepared in accordance with a format defined by a regulatory authority of a country in which the present invention is practiced (e.g., Ministry of Health, Labour and Welfare in Japan, Food and Drug Administration (FDA) in the U.S.), explicitly describing that the instructions are approved by the regulatory authority. The instructions are so-called package insert and are typically provided in paper media; however, the instructions are not limited thereto, and may be provided in the form of electronic media (e.g., web sites provided on the Internet and electronic mails).

In the therapy of the present invention, two or more pharmaceutical agents may be used as required. When two or more pharmaceutical agents are used, these agents may have similar properties or may be derived from similar origins, or alternatively, may have different properties or may be derived from different origins. The method of the present invention can be used to obtain information about a disease level for a method for administering such two or more pharmaceutical agents.

Culturing methods used in the present invention are described and supported in, for example, "Animal Culture Cell Manual, Eeno et al., eds., KYORITSU SHUPPAN Co., Ltd., 1993, the entirety of which is hereby incorporated by reference.

### (Gene therapy)

In certain embodiments, a nucleic acid including a sequence encoding an antibody or a functional derivative thereof is administered for the purpose of gene therapy for treating, inhibiting or preventing a disease or disorder related to abnormal expression and/or activity of the polypeptide of the present invention. Gene therapy refers to a therapy performed by administering a nucleic acid, which has been expressed or is capable of being expressed, to subjects. In this embodiment of the present invention, a nucleic acid produces a protein encoded thereby and the protein mediates a therapeutic effect.

Any method available in the art for gene therapy may be used in accordance with the present invention. Illustrative methods are described below.

See the following general review for a method for gene therapy: Goldspiel et al., Clinical Pharmacy 12:488-505 (1993); Wu and Wu, Biotherapy 3: 87-95 (1991); Tolstoshev, Ann. Rev. Pharmacol. Toxicol. 32: 573-596 (1993); Mulligan, Science 260: 926-932 (1993); and Morgan and Anderson, Ann. Rev. Biochem. 62:191-217 (1993); and May, TIBTECH 11(5): 155-215(1993). Generally known recombinant DNA techniques used for gene therapy are described in Ausubel et al. (eds.), Current Protocols in Molecular Biology, John Wiley & Sons, NY (1993); and Kriegler, Gene Transfer and Expression, A Laboratory Manual, Stockton Press, NY (1990).

### (Demonstration of therapeutic activity or preventive activity)

The compounds or pharmaceutical compositions of the present invention are preferably tested in vitro, and then in vivo for the desired therapeutic or preventive activity, prior to use in humans. For example, in vitro assays to demonstrate the therapeutic or preventive utility of a compound or pharmaceutical composition include the effect of a compound on a cell line, or a patient tissue sample. The effect of the compound or composition on the cell line and/or tissue sample can be determined utilizing techniques known to those skilled in the art (including, but not limited to, cell lysis assays). In accordance with the present invention, examples of the in Vitro assays which can be used to determine whether or not administration of a specific compound is indicated include in Vitro cell culture assays. In this assay, a patient tissue sample is grown in a culture, and exposed to a compound or otherwise a compound is administered to the patient tissue sample, and the effect of such a compound on the tissue sample is observed.

### (Administration for therapy/prevention and composition)

The present invention provides methods for treatment, inhibition, and prevention by administration to a subject an effective amount of a component or pharmaceutical composition including the enhancer for a promoter of the present invention. In a preferred aspect, the component including an enhancer for a promoter may be substantially purified (for example, including the state where the effects are restricted, or a substance causing undesirable side effect is substantially free). Subjects may preferably be animals including, but not limited to, cattle, pigs, horses, chickens, cats, dogs, and the like, and preferably mammals, and most preferably humans.

When the nucleic acid molecule or polypeptide of the present invention is used as a pharmaceutical agent, such a composition may further contain a pharmaceutically acceptable carrier and the like. Examples of the pharmaceutically acceptable carrier contained in the pharmaceutical agent of the present invention include any substance known in the art.

Examples of such a suitable formulation material or the pharmaceutically acceptable carrier include, but are not limited to, antioxidants, preservatives, colorants, flavoring agents, diluents, emulsifiers, suspending agents, solvents, fillers, bulky agents, buffers, delivery vehicles, diluents, excipients and/or pharmaceutical adjuvants. Typically, the pharmaceutical agent of the present invention is administered in the form of a composition including an isolated multipotential stem cell or a variant or derivative thereof, with at least one physiologically acceptable carrier, excipient or diluent. For example, an appropriate vehicle may be an injection solution, a physiological solution, or an artificial cerebrospinal fluid. Such a vehicle can be supplemented with other substances commonly used for compositions for parenteral delivery.

The acceptable carriers, excipients or stabilizers used in the present specification are nontoxic to recipients and are preferably inert at the dosages and concentrations to be employed, and examples thereof include, but are not limited to, phosphate, citrate, or other organic acids; ascorbic acid, (alpha-tocopherol; low molecular weight polypeptides; proteins (e.g., serum albumin, gelatin, and immunoglobulins); hydrophilic polymers (e.g., polyvinylpyrrolidone); amino acids (e.g., glycine, glutamine, asparagine, arginine and lysine); monosaccharides, disaccharides, and other carbohydrates (including glucose, mannose, and dextrins); chelating agents (e.g., EDTA); sugar alcohols (e.g., mannitol and sorbitol); salt-forming counterions (e.g., sodium); and/or nonionic surfactants (e.g., Tween, pluronics and polyethylene glycol (PEG)).

Examples of the appropriate carriers include neutral buffered saline or saline mixed with serum albumin. Preferably, the product is formulated as a lyophilizate using appropriate excipients (e.g., sucrose). Other standard carriers, diluents, and excipients may be included as desired. Other exemplary compositions include Tris buffer with pH 7.0 to 8.5, or acetate buffer with pH 4.0 to 5.5, which may further include sorbitol or a suitable alternative thereof.

The pharmaceutical agent of the present invention may be administered orally or parenterally. AlternatiVely, the pharmaceutical agent of the present invention may be administered intravenously or subcutaneously. When systemically administered, the pharmaceutical agent for use in the present invention may be in the form of a pyrogen-free, pharmaceutically acceptable aqueous solution. The preparation of such pharmaceutically acceptable compositions can be easily performed by those skilled in the art in consideration of pH, isotonicity, stability and the like. In the present specification, administration methods may be oral administration, and parenteral administration (e.g., intravenous administration, intramuscular administration, subcutaneous administration, intradermal administration, mucosal administration, intrarectal administration, intravaginal administration, topical administration to an affected site, skin administration). A dosage formulation for such administration may be provided in the form of any formulation. Example of the form of formulation include liquid formulations, injections, and sustained preparations.

The pharmaceutical agent of the present invention may be prepared and stored in the form of lyophilized cake or aqueous solutions by mixing a sugar chain composition having the desired degree of purity with, according to necessity, physiologically acceptable carriers, excipients, or stabilizers (see Japanese Pharmacopoeia 14th edition, or a supplement thereto or the latest edition thereof, Remington's Pharmaceutical Sciences, 18th Edition, A. R. Gennaro, ed., Mack Publishing Company, 1990; and the like).

The amount of a sugar chain composition used in the treatment method of the present invention can be easily determined by those skilled in the art in view of the purpose of use, the target disease (kind, severity, etc.), the patient's age, weight, sex, and past history, the form and kind of a cell, or the like. The frequency of subjecting a subject (patient) to the treatment method of the present invention can also be easily determined by those skilled in the art in view of the purpose of use, the target disease (kind, severity, etc.), the patient's age, size, weight, and past history, the progression of the therapy, and the like. Examples of the frequency of administration include once per day to once per several months (e.g., once per week to once per month). Preferably, administration is preferably performed once per week to once per month with reference to the progression.

### (General techniques used in this specification)

Techniques used in the present specification uses well-known and routinely used techniques in the fields of sugar chain science, microfluidics, microfabrication, organic chemistry, biochemistry, genetic engineering, molecular biology, microbiology, genetics, and their relevant fields, which are within the technical scope of the present invention unless otherwise specified. The techniques are sufficiently well described in documents described below and other documents cited in other places in the present specification.

Microfabrication is described in, for example, Campbell, S.A. (1996). The Science and Engineering of Microelectronic Fabrication, Oxford University Press; Zaut, P.V. (1996). Micromicroarray Fabrication: a Practical Guide to Semiconductor Processing, Semiconductor Services; Madou, M.J. (1997). Fundamentals of Microfabrication, CRC1 5 Press; Rai-Choudhury, P. (1997). Handbook of Microlithography, Micromachining & Microfabrication: Microlithography; and the like, the relevant portions of which are herein incorporated by reference.

Molecular biology procedures, biochemistry procedures, microbiology procedures and sugar chain scientific procedures used in the present specification are well-known and routinely used in the art, and are described in, for example, Maniatis, T. et al. (1989). Molecular Cloning: A Laboratory Manual, Cold Spring Harbor and its 3rd Ed. (2001); Ausubel, F.M. et al. eds, Current Protocols in Molecular Biology, John Wiley & Sons Inc., NY, 10158 (2000); Innis, M.A. (1990). PCR Protocols: A Guide to Methods and Applications, Academic Press; Innis, M.A. et al. (1995). PCR Strategies, Academic Press; Sninsky, J.J. et al. (1999). PCR Applications: Protocols for Functional Genomics, Academic Press; Gait, M.J. (1985), Oligonucleotide Synthesis: A Practical Approach, IRL Press; Gait, M.J. (1990). Oligonucleotide Synthesis: A Practical Approach, IRL Press; Eckstein, F. (1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; Blackburn, G.M. et al. (1996). Nucleic Acids in Chemistry and Biology, Oxford University Press; Hermanson, G.T. (1996). Bioconjugate Techniques, Academic Press; Method in Enzymology 230, 242, 247, Academic Press, 1994; Special issue, Experimental Medicine "Experimental method for Gene Introduction & Expression Analysis)", YODOSHA Co., Ltd., 1997; and the like, and the relevant portions (or possibly the entirety) of which are herein incorporated by reference.

### (Description of preferred embodiments)

Hereinafter, preferred embodiments will be described; however, the embodiments are provided for illustrative purposes of the present invention. Accordingly, it should be understood that the scope of the present invention is not limited to the preferred embodiments. It should be understood by those skilled in the art that variations and modifications can be easily made within the scope of the present invention with reference to the following preferred embodiments.

### (Enhancer for promoter)

In an aspect, the present invention provides an enhancer for a promoter, which comprises an intron sequence for a major immediate early gene (MIE) of human herpes virus-6 (HHV-6) or a fragment of the intron sequence. In particular, it has been found that this enhancer has unexpectedly enhanced the ability of a viral promoter such as an HHV-6B MIE promoter or the like.

It has been found that the HHV-6B MIE promoter, with an HHV7 MIE promoter, and an HHV7 U95 promoter, is unexpectedly enhanced in selectivity to lymphocytes in comparison to HCMV IE promoters. It is understood that the enhancer of the present invention can further enhance such selectivity. Further, it has been found that in adhesive cells (293 cells, Vero cells and the like), the MIE promoter only showed one hundredth the activity of that of HCMV IE promoter, whereas in lymphoid cells such as SupT1 and U937, a several fold increase in expression efficiency is obtained. Such a high level of selectivity or specificity accounts for capability of application to DNA vaccines, and particularly to the development of a pharmaceutical agent that targets lymphocytes as gene therapy. In this regard, it is understandable that the enhancer of the present invention can further enhance such an effect. Moreover, in an expression system in vivo, since activities are diminished even in the case of CMV promoters which have potent activity, due to the action of methylase, it is understood that the enhancer for a promoter of the present invention may be used to secure an amount of expression in vivo in blood cells or lymphocyte cells. In genetic diseases, gene therapy for cancer, or the like, retrovirus vectors are generally used; however, since LTR activity is not so potent as a promoter, the introduction of the enhancer for a promoter of the present invention into the upstream of the gene to be expressed allows potent expression in blood cells. The present invention is useful in gene therapy targeting blood cell diseases such as leukemia. Furthermore, RNAi is used as a method for knocking out gene expression, and the enhancer for a promoter of the present invention is used as an enhancer for a promoter of hair-pin type RNA expression vectors, thereby allowing more efficient effects of inhibition of expression in the blood cells. Macrophages, dendritic cells or the like are purified from native peripheral blood using flow cytometry, and these cells are transfected with plasmids constructed so as to express cancer specific antigen, tumor necrosis factor (TNF) gene and the like under controlling the enhancer for a promoter of the present invention, and reintroduced to the original body after confirmation of expression of a cancer antigen, thereby practicing the gene therapy of cancer as a result of efficient activation of cancer antigen specific CTL via Class I-HLA.

In one embodiment, the promoter to be used in the present invention includes a length of at least 8 contiguous nucleotide sequences, amongst the sequence set forth in SEQ ID NO: 1. Preferably, the promoter of the present invention includes at least the R3 region or the functional variant thereof, amongst the sequence set forth in SEQ ID NO: 1. More preferably, the promoter of the present invention includes a sequence of at least -574 to -427 based on a transcription initiation point; more preferably, a sequence of at least - 1051 to -427 based on a transcription initiation point, amongst the sequence set forth in SEQ ID NO: 1.

In one embodiment, the intron sequence of the present invention includes a sequence (SEQ ID No. 2) of at least -12 to +1292 based on a transcription initiation point of IE1 included in the above 6MIE sequence, or a fragment of the intron sequence. It has not been conventionally known that the intron sequence of the present invention has an enhancer effect, and the intron sequence of the present invention has not also been conventionally known as a viral promoter, and thus it is entirely unexpectable.

In a preferred embodiment, the intron sequence of the present invention includes a sequence (SEQ ID No. 3) of -12 to +262 based on a transcription initiation point of IE1 included in the above 6MIE sequence. More preferably, the intron sequence of the present invention is comprised of the sequence (SEQ ID No. 3) of -12 to +262 based on the transcription initiation point of IE1 included in the 6MIE sequence. It is understood that such a sequence may have one or more, or at least one nucleotide substitution, addition and/or deletion as long as it has the enhancer effect.

In one embodiment, a promoter as a target of the enhancer of the present invention includes: (a) a polynucleotide having the base sequence set forth in SEQ ID NO: 1, or a base sequence corresponding thereto or a fragment sequence thereof; (b) a polynucleotide of an allelic variant of the base sequence set forth in SEQ ID NO: 1 or a base sequence corresponding thereto or a fragment sequence thereof; (c) a polynucleotide which hybridizes the polynucleotide of any of (a) and (b) under stringent conditions and has a biological activity; or (d) a polynucleotide of any of (a) to (c) or a polynucleotide which contains the base sequence with at least 70% identity to a complement sequence thereof, and has a biological activity. Here, the biological activity may be a promoter activity and/or an enhancer activity, but is not limited thereto. The promoter activity and the enhancer activity can be determined using techniques well-known in the art. Such techniques are also described in the present specification and illustrated in examples.

In one preferred embodiment, a promoter as a target of the enhancer of the present invention may include substitution, addition, or deletion in the above (a) to (d). The numbers of such substitutions, additions, and deletions may be preferably limited to, for example, 50 or less, 40 or less, 30 or less, 20 or less, 15 or less, 10 or less, 9 or less, 8 or less, 7 or less, 6 or less, 5 or less, 4 or less, 3 or less, or 2 or less. The smaller the numbers of substitutions, additions, and deletions is preferred. However, the larger the number of substitutions, additions, and deletions is allowable as long as the promoter retains its biological activities (preferably, the promoter has similar or substantially the same activities as those of HHV-6B MIE promoter).

In a preferred embodiment, identity to one of polynucelotides of any one of the above (a) to (d) or a complementary sequence thereof may be at least about 80%, more preferably at least about 90%, further preferably at least about 98%, and most preferably at least about 99%.

In another preferred embodiment, a promoter as a target of the present invention includes a sequence of at least -382 to -983 based on a transcription initiation point of IE1 included in the above 6MIE sequence or a fragment of the sequence. Although not wishing to be bound by theory, it is because a sequence with a remarkable enhancer effect of the present invention can be found in this region. Furthermore, it should be understood that a region where such an enhancer effect can be found is not limited to the above region.

### (Promoter)

In another aspect, the present invention provides a promoter with improved activity, including: an intron sequence for a major immediate early gene (MIE) of human herpes virus-6 (HHV-6) (particularly, HHV-6B) or a fragment of the sequence; and a promoter.

In the present specification, the "promoter with improved activity," of the present invention can be understood as any promoter with improved activity higher than that of the promoter used alone.

In one embodiment, it is understood that an intron sequence for a major immediate early gene (MIE) of human herpes virus 6 (HHV-6), which is used in the promoter with improved activity of the present invention, or a fragment of the sequence can employ any embodiment described in the above section "Enhancer for promoter".

In one embodiment, furthermore, it is understood that a promoter to be used for the promoter with improved activity of the present invention can employ any embodiment described in the above section "Enhancer for promoter".

In a preferred embodiment, the promoter with improved activity of the present invention includes the sequence (SEQ ID No. 2) of at least -12 to +1292 of IE1 included in the 6MIE sequence, or a fragment of the sequence, and an MIE promoter. Although not wishing to be bound by theory, this is because the use of such a combination provides a promoter with remarkably improved promoter effect, compared with the conventional MIE promoter.

Here, preferably, the MIE promoter used in the present invention includes a sequence (SEQ ID No. 4) of at least -382 to -983 based on a transcription initiation point of IE1 included in the above 6MIE sequence, or a fragment of the sequence.

### (Gene construct)

In another aspect, the present invention provides a construct for enhancing expression of a gene in a cell, including an intron sequence for a major immediate early gene (MIE) of human herpes virus-6 (HHV-6) or a fragment of the sequence and a promoter.

In one embodiment, it is understood that the intron sequence for a major immediate early gene (MIE) of human herpes virus 6 (HHV-6) or a fragment of the sequence can employ any embodiment described in the above section "Enhancer for promoter".

Furthermore, in one embodiment, it is understood that the promoter used in the construct of the present invention can also employ any embodiment described in the above section "Enhancer for promoter".

In a preferred embodiment, the construct of the present invention is a construct for enhancing expression of a gene in a cell, and includes the sequence (SEQ ID No. 2) of at least - 12 to +1292 based on a transcription initiation point of IE1 included in the above 6MIE sequence, or a fragment of the sequence, and an MIE promoter. Although not wishing to be bound by theory, this is because the use of such a combination provides a construct with remarkably improved promoter effect, compared with the conventional MIE promoter.

In one embodiment, cells as a target of the construct of the present invention are blood cells, particularly cells of T-lymphocyte or adherent line. Examples of the cells of T-lymphocyte line include Molt-3, Jurkat, and SupT1. Examples of the cells of adherent line include MRC-5, MeWo, and U373.

In one embodiment, the nucleic acid construct of the present invention includes a sequence encoding a foreign gene derived from a source different from the promoter and the enhancer thereof which are used in the present invention, where the sequence is operatively linked to a sequence of the promoter used in the present invention.

Examples of such a foreign gene include, but are not limited to, one encoding an RNAi molecule, a pharmaceutical agent, a recessive gene to be deleted, or a selective marker.

Preferably, the selective marker to be used in the present invention may be one that allows selection in a medium of a host in which the nucleic acid construct of the present invention is introduced. For example, the selective marker may be one that allows visual selection in a host in which the nucleic acid construct is introduced, and examples thereof include hypoxanthine guanine phosphoribosyl transferase (hprt) and green fluorescent protein (GFP), cyan fluorescent protein (CFP), yellow fluorescent protein (YFP), and red fluorescent protein (dsRed).

Preferably, it is advantageous that the selective marker included in the nucleic acid construct of the present invention does not substantially exhibit toxicity against the host in which the nucleic acid construct of the present invention is introduced. This is because, no adverse effect is preferred when the present invention is used for therapeutic or preventive purposes.

Examples of those to be included in the nucleic acid construct of the present invention include recessive genes to be deleted. Here, a recessive gene to be deleted refers to any recessive gene which exhibits a diseased condition when deleted. Examples of such a recessive gene include ADA gene (which is related to severe combined immunodeficiency (SCID)), PNP gene (which is related to severe combined immunodeficiency (SCID)), γc chain gene (which is related to severe combined immunodeficiency (SCID)), TAP gene (which is related to MHC I deficiency), MHC II gene (which is related to MHC II deficiency), X-linked WASP (which is related to Wiskott-Aldrich syndrome), CD40 ligand (which is related to X-linked high IgM syndrome), PI3K-like gene (which is related to ataxia telangiectacia), and DNA helicase (which is related to Bloom's syndrome).

In a preferred embodiment, pharmaceutical agents to be included in the nucleic acid construct of the present invention may be proteinous pharmaceutical agents such as cytokines, chemokines, growth factors, protein hormones, and peptide hormones such as IFN-α, IFN-γ, IL-2, IL-12, G-CSF, and GM-CSF.

In another aspect, the present invention provides an expression vector including the nucleic acid construct of the present invention. Such an expression vector includes elements essential to expression, which may not exist in the nucleic acid construct of the present invention, for example, terminators and enhancer sequences other than those of the present invention in an operatively linked manner, which allow expression in the host. In another preferred embodiment, the selective markers may be immortalizing genes (for example bcl-2). Alternatively, the selective markers may be hypoxanthine phosphoribosyl transferase (HPRT), a gene encoding a toxic product, a toxic gene product that is active depending on a condition in combination with a suicide substrate (for example, herpes simplex virus thymidine kinase (HSV-TK) in combination with ganciclovir) .

In another aspect, the present invention provides a cell including the construct of the present invention. Such a cell, in the case of a lymphocyte, promotes the expression of a protein encoded by a foreign gene.

Preferably, it is advantageous that the cell of the present invention is heterogenous to the promoter sequence used in the present invention. It can be said that it is one of surprising effects to have a promoter activity even if the cell is heterogenous. A method for introducing the nucleic acid molecule of the present invention into a cell is well-known in the art, and described in detail in the above section. Alternatively, such a cell may be identified by screening a cell containing the nucleic acid molecule in a sample including the cell. The cell containing the nucleic acid molecule of the present invention may preferably be in an undifferentiated state. The cell expressing the nucleic acid molecule of the present invention is usually in an undifferentiated state. Therefore, a cell into which such a nucleic acid molecule has been introduced so as to be expressed in a controllable manner can control the undifferentiated state. Alternatively, such a cell may be used to produce the nucleic acid molecule of the present invention in a large amount. Such production methods are well-known in the art and are described in the documents described in the present specification.

In another aspect, the present invention provides a tissue including the construct of the present invention. Such a nucleic acid sequence is preferably operatively linked to a control sequence. Such a tissue may be an animal tissue, or a tissue of a different organism such as a plant. Alternatively, such a tissue may be used to produce the nucleic acid molecule of the present invention in a large amount. Such production methods are well-known in the art and are described in the documents described in the present specification.

In another aspect, the present invention provides an organ including the construct of the present invention. Such a nucleic acid sequence is preferably operatively linked to a control sequence. Such an organ or body organ may be an animal organ or body organ, or an organ or body organ of a different organism such as a plant. Alternatively, such an organ or body organ may be used to produce the nucleic acid molecule of the present invention in a large amount. Such production methods are well-known in the art and are described in the documents described in the present specification.

In another aspect, the present invention provides an organism including the construct of the present invention. Such an organism may be an animal or a different organism such as a plant. Alternatively, such an organism may be used to produce the nucleic acid molecule of the present invention in a large amount. Such production methods are well-known in the art and are described in the documents described in the present specification.

### (Method for enhancing expression of gene in cell)

In another aspect, the present invention provides a method for enhancing expression of a gene in a cell, including the steps of:
1) generating a construct including the enhancer for a promoter of the present invention and a promoter, where the gene is arranged so as to be operatively linked to a sequence;
2) introducing the construct into the cell; and
3) culturing the cell under conditions for expressing the gene.

Alternatively, the present invention provides a kit for enhancing expression of a gene in a cell, including:
1) a construct including the enhancer for a promoter of the present invention and a promoter, where the gene is arranged so as to be operatively linked to a sequence;
2) a reagent for introducing the construct into the cell; and
3) a means for culturing the cell under conditions for expressing the gene. It is understood that the enhancer for a promoter, promoter, construct, cell, and the like used in this method can employ any form described in the sections "Enhancer for promoter", "Promoter", and "Construct". The reagent for introducing the construct into a cell is known in the art, and any of them may be used. A culturing means is also known in the art, and any of them may be used.

### (Use)

In another aspect, the present invention provides use of a sequence (SEQ ID No. 2) of at least -12 to +1292 based on a transcription initiation point of IE1 included in the 6MIE sequence, or a fragment of the sequence, as an enhancer for a promoter. Here, it is understood that the enhancer for a promoter can employ any form described in the section "Enhancer for promoter".

In one aspect, such a use form may be a pharmaceutical agent.

Therefore, in another aspect, the present invention provides a pharmaceutical composition including the enhancer for a promoter of the present invention, a promoter, and an antigen-encoding sequence. Here, the antigen may be any protein desired to raise an immune response in a host. Examples of such an antigen include, but are not limited to, cancer antigens. Therefore, the pharmaceutical composition of the present invention may preferably be a DNA vaccine.

In another aspect, the enhancer for a promoter of the present invention provides a pharmaceutical composition for treating a disease, disorder or condition in which a lymphocyte-specific treatment is desired, the composition including the enhancer for a promoter of the present invention, a promoter, and a nucleic acid sequence for the treatment. Here, the target of the pharmaceutical composition may appropriately be any of diseases, disorders, conditions and the like desired to have lymphocyte specific treatment, and examples thereof include acquired immunodeficiency syndromes. Examples of the acquired immunodeficiency syndromes include, but are not limited to, severe combined immunodeficiency (SCID), MHC I deficiency, MHC II deficiency, Wiskott-Aldrich syndrome, X-linked high IgM syndrome, ataxia telangiectacia, and Bloom's syndrome. Although not wishing to be bound by theory, acquired immunodeficiency syndrome is caused by some deficiency in a recessive gene (which is herein also referred to as a recessive gene to be deleted). It is possible to carry out somatic gene therapy in which this gene to be deleted is introduced to bone marrow cells taken from a patient then the cells are reintroduced into the patient. In this case, the HHV-6B MIE promoter to which the enhancer for a promoter of the present invention may target is utilized to expect an increase in efficiency of gene expression in a cell differentiated into T cell, macrophage and the like. Introduction of such a gene construct can be carried out, for example, by using a retrovirus and the like.

In a preferred embodiment, the nucleic acid sequences for the treatment to be used in the present invention include nucleic acid sequences encoding cytokines, nucleic acid sequences encoding chemokines, nucleic acid sequences encoding growth factors, nucleic acid sequences encoding protein hormones, nucleic acid sequences encoding peptide hormones, ribozymes, and RNAi.

In another aspect, the present invention provides use of the enhancer for a promoter of the present invention in production of a pharmaceutical composition for treating a disease, disorder, or condition.

Reference documents such as scientific references, patents and patent applications cited in the present specification are incorporated by reference in their entirety in the present specification to the same extent as that each is specifically described.

As described above, the present invention has been described for easy understanding by showing preferred embodiments. The present invention will be described below based on examples, but the aforementioned description and the following examples are provided only for illustration, and are not provided for the purpose of limiting the present invention. Therefore, the scope of the present invention is not limited to embodiments or examples which are specifically described in the present specification, and is limited only by claims.

### EXAMPLES

Handling of animals used in the following examples was in accordance with the provisions set forth in Osaka University.

### (Example 1)

It was confirmed that the activity of a major immediate early gene (MIE) promoter of human herpes virus 6 (HHV-6) (6MIEp) was higher than that of the IE promoter (SEQ ID NO. 35) of cytomegalovirus (CMV) in T cells. In this example, a promoter with a prolonged 3' end of HHV-6B MIE promoter was prepared, and investigation was performed whether a similar effect could be obtained with respect to the intron of IE1.

### (Materials and methods)

The upstream region of MIE gene of HHV-B strain HST was amplified using various primers described below (Fig. 1).
full Fw primer: 5'-TCTCTCGAGAGTTAAAGATCAGCGGGTAC-3'(SEQ ID NO. 7);
-d1 Fw primer: 5'-AGTCGGTACCGGCGAATGAGAACTCTAAAAGCTC-3' (SEQ ID NO. 8);
-d2 Fw primer: 5'-AGTCGGTACCTACTGTGGTTGGGGTCTTTCCTAC-3' (SEQ ID NO. 9);
-d3 Fw primer: 5'-AGTCGGTACCACATTCCTGTTTCATGATGTGTAGC-3' (SEQ ID NO. 10);
-d4 Fw primer: 5'-AGTCGGTACCTCCTGTTTTTGAGTAAGATATGAC-3' (SEQ ID NO. 11) ;
-d5 Fw primer: 5'-AGTCGGTACCAGCTAATTTCCATTCCATATTTGTC-3'(SEQ ID NO. 12);
-d6 Fw primer: 5'-AGTCGGTACCTACAGCGATTGGCTCCTTCATCCTC-3' (SEQ ID NO. 13);
6MIEp Rv primer: 5'-AGTCCTCGAGCACTGAACTGGCTGTAACTTCTGC-3'(SEQ ID NO. 14);
6MIEp-inl Rv primer: 5'-TCTAAGCTTCAGCAATCCAATAATTGATG-3'(SEQ ID NO. 15);
6MIEp-in2 Rv primer: 5'-CATAAGCTTGCATACGTTCCTCATTGGAT-3'(SEQ ID NO. 16);
6MIEp-in3 Rv primer: 5'-CATAAGCTTCCAAAGTTTTGAATTCTTCA-3'(SEQ ID N0. 17);
6MIEp-in4 Rv primer: 5'-CATAAGCTTTTTGGATGCAAGTGCCAACG-3'(SEQ ID NO. 18).

Variants of the obtained various promoter were inserted into pGL3 basic to construct reporter plasmids, respectively. Various kinds of culture cells (Molt-3, MRC-5, Jurkat, MeWo, SupTl, and U373) were transfected with the constructed reporter plasmids, and their luciferase activities were then measured.

Specifically, on a day before the transfection, adherent line cells were seeded 1 x 10⁵ cells/well to a 24 well plate and then incubated overnight at 37°C under 5%CO₂. Floating line cells were seeded 4 x 10⁵ cells/well to a 24 well plate immediately before the transfection. Using Lipofectamine 2000 (Invitrogen), various kinds of culture cells (Melt-3, MRC-5, Jurkat, MeWo, SuPT1, and U373) were transfected with 1 µg of various constructed reporter plasmids or empty vectors together with 0.25 µg of Renilla luciferase expression plasmid (pRL-TK) for correcting transfection efficiency. After 24 hours from the transfection, the cells were collected. The cells were lysed with Dual-luciferase reporter assay system (Promega). The amount of luminescence due to firefly luciferase and Renilla luciferase in the cell lysate were measured, respectively. Here, the measurement was conducted with a multi-mode microplate reader LB941 (Berthold). The amount of luminescence due to firefly luciferase obtained by measurement was divided by the amount of luminescence due to Renilla luciferase to correct the transfection efficiency. Furthermore, the resulting value was divided by the amount of luminescence of the empty vector to obtain a relative value of the promoter activity to the empty vector.

### (Result)

As a result of the reporter assay with luciferase, any of promoters extending to intron 1 showed increased activities in any cells. In contrast, however, promoters extending to introns 2 to 4 showed a decrease in activity (Fig. 2). Enhancement of promoter activity with addition of intron 1 was also found in any promoter defective mutants (Fig. 3).

### (Consideration)

The results of the reporter assay of promoters reveal that the addition of intron 1 causes an increase in activity. The T-cell line shows higher activity than the CMVIE promoter. In addition, some of adherent line cells, such as MeWo cells and MRC-5 cells, show an increase in activity up to the same level as that of the CMVIE promoter. It is supposed that such an increase in activity may be useful in foreign gene expression.

### (Example 2)

### (Examination of poly-A addition reaction derived from herpes virus)

A polyA tail is added to the 3'-end of mRNA. The polyA tail is considered to stabilize mRNA and promote protein expression. The polyA tail targets a sequence of AAUAAA on the 3' region of mRNA and is added thereto by polyA polymerase. A region that contains a polyA addition sequence derived from herpes virus is inserted into the downstream of a gene that encodes a protein of interest, attempting to protein expression.

### (Materials and methods)

Regions containing portions considered to be polyA addition sequences derived from HHV-6B, U90, and U100 illustrated below were amplified by PCR, respectively. These were inserted into the downstream of DsRed2 gene of pBlueScriptSK(-)-DsRed2 vector (Clontech). Then, 6MIE was used as a promoter to construct plasmids pBleuScriptSK(-)-6 MIE-DsRed2-U90pA (SEQ ID NO. 39) and pBlueScriptSK(-)-6 MIE-DsRed2-U100pA (SEQ ID NO. 40).

The cloned U90pA sequence (complementary strand of 134474 to 134679 of HHV-6B HST genome, the underlined portion is assumed as poly-A addition sequence) is as follows: 
5′-
TTAGAAATTACATAAGCAAATGTAACTTTTTCTATTATGTAAAACCTCAGCAAACATGTGAT
TTCTCAAAGGAATTTATTTTCAATATCACCTTACAAATAATAAAAGTCATACAGACATTGC
TTTCTCTTTTTAATTCCAAGTCATGTACATAAACTGACTTATAAGTCATCATACAATATTTC
CATAAGTTAATTCCAGCTTT-3′ (SEQ ID NO. 37)
 The cloned 100pA sequence (complementary strand of 148150 to 148301 of HHV-6B HST genome, the underlined portion is assumed as poly-A addition sequence) is as follows: 
5'-
CCCTCAGACCCTACGTTGCCCTCACCTTATGGCAACGGACATTATTAAAATAAAAAATTAC
TGAAAGAGAGTCAGAAATTGTGTCACATGTTATTTTATTAAATCTTACTGAACTTTGTCCTT
TGTCCTTACAAACCTTCCCTATCACAATAC-3' (SEQ ID NO. 38)
 Various kinds of culture cells (293T, MeWo, MRC5, and HeLa) were transfected with the obtained plasmids, and expression of DeRed was then confirmed under fluorescence microscope.

Specifically, on the day before the transfection, various kinds of culture cells (293T, MeWo, MRC5, and HeLa) were seeded 2 x 10⁵ cells/well to a 6-well plate and then incubated overnight at 37°C under 5%cho2• Transfection of 1 µg pBlueScript plasmid, to which the above expression cassette had been inserted, was conducted using Lipofectamine 2000 (Invitrogen). After overnight incubation at 37°C under 5%CO₂, DsRed expression was confirmed using fluorescence microscope.

### (Example 3: Construction of specific deletion system)

Knocking out of gene expression in blood cells was conducted using an IE promoter and an RNAi method together with the enhancer for a promoter of the present invention. IE promoters are advantageous for analysis since they are expressed in blood cells in a large amount.

### 1) Preparation of cells (in the case of macrophages)

Healthy human peripheral blood was collected, separated and purified by density gradient using Ficoll/Hypaque. The PBMCs were cultured in AIM V serum medium (Life Technologies) supplemented with M-CSF (R&D systems, 100U/ml). The medium was exchanged every three days, and macrophages at Day 6 or 7 after culture were used for experiments.

### 2) Production of siRNA expression retrovirus vector

In order to express hair-pin type RNA; a synthetic oligo-DNA including "a sense strand target sequence", "a loop sequence", "an antisense strand target sequence" and "a terminator sequence" were produced. Such a sense strand target sequence, loop sequence, antisense target sequence, terminator sequence may be made using a well-known technique in the art. Those skilled in the art can understand that when actually using these, an appropriate sequence may be employed depending on the actual situation.

The above-mentioned DNA was incorporated into a plasmid vector in which the oligo-DNA was linked to the downstream of the IE sequence, gag, pol, and env which are necessary for replication of a retrovirus were deleted, and Neo^{R} gene was included, by making use of restriction enzyme sequences and the like. Plasmid vector produced (10 µl) was added to 100 µl of competent cells and transformation was conducted and the resultant was cultured for 16 hours at 37°C after plating into LBAmp plate. Colonies obtained by the transformation were cultured on LBApm liquid medium at 37°C for 16 hours, and plasmids were extracted and purified using conventional methods from the culture solution.

Retrovirus packaging cells expressing gag, pol and env were plated on a disc with a 10-cm diameter, and transfection reagent was opened to transfect the plasmid (10 µg). After 24 to 48 hours, the cells were subjected to serial dilution into a G418 containing medium (500 µg/ml) and passaged.

Every three to four days, the G418 medium was exchanged and cultured for about two weeks in total. The colonies were recovered and at the time where growth was found at a confluent level on a six-well plate, the medium was changed to a G418 free medium and the supernatant was recovered 24 hours later. Subsequently, cells were stocked.

Retrovirus vectors included in the supernatant were subjected to serial dilution, and infected into NIH/3T3 cells, and colonies grown were counted to calculate the infection value.

### 3) Gene introduction experiment using retrovirus vectors

Retrovirus vectors were infected with blood cells such as macrophages prepared in 1). Immediately after washing, the cells were plated so as to be 0.5 to 2.5 x 10⁴ cells/cm² in a plate. Twenty four hours after the infection, the medium was exchanged with a G418 containing medium, and every three to four days, the medium was exchanged. About two weeks later, gene introduced cells were obtained. The cells were used to confirm the amount of expression of the knocked out gene of interest.

These experiments were conducted to actually confirm that after gene introduction, lymphocyte specific expression of a foreign gene was knocked out with the promoter used in the present invention.

### (Example 4: Specific expression)

Instead of the RNAi of Example 3, a nucleic acid molecule encoding a gene (e.g., cytokines such as TGFβ) desired for expression was introduced.

As a result, by conducting the same experiments as in Example 3, after gene introduction, it was confirmed that the enhancer for a promoter of the present invention actually induced the specific expression of a foreign gene in such a manner that the promoter activity was enhanced.

As described above, the present invention has been exemplified using preferred embodiments of the present invention; however, it is understood that the scope of the present invention should be construed only by the claims. It is understood that a content of patents, patent applications and references cited in the present specification should be incorporated into the present specification by reference as if the content itself is specifically described in the present specification.

### INDUSTRIAL APPLICABILITY

The present invention has provided an enhancer for a viral promoter. The enhancer for a promoter of the present invention is useful in a method and pharmaceutical agent for effectively preventing or treating immune diseases such as acquired immunodeficiency syndromes. The present invention is also useful in the techniques for efficiently conducting gene therapy.

### SEQUENCE LISTING DESCRIPTION

SEQ ID N0. 1 is a sequence of an HHV-6B MIE promoter.
SEQ ID NO. 2 is a sequence of -12 to +1292 based on a transcription initiation point of IE1 contained in MIE of HHV-6B.
SEQ ID NO. 3 is a sequence of -12 to +262 based on a transcription initiation point of IE1 contained in MIE of HHV-6B.
SEQ ID NO. 4 is a sequence of -382 to -983 based on a transcription initiation point of IE1 contained in MIE of HHV-6B.
SEQ ID NO. 5 is a sequence of -530 to +383 based on a transcription initiation point of IE1 contained in MIE of HHV-6B.
SEQ ID NO. 6 is a sequence of -1007 to +383 based on a transcription initiation point of IE1 contained in MIE of HHV-6B.
SEQ ID NO. 7 is a sequence of full Fw primer used in Example 1.
SEQ ID NO. 8 is a sequence of -d1 Fw primer used in Example 1.
SEQ ID NO. 9 is a sequence of -d2 Fw primer used in Example 1.
SEQ ID NO. 10 is a sequence of -d3 Fw primer used in Example 1.
SEQ ID NO. 11 is a sequence of -d4 Fw primer used in Example 1.
SEQ ID NO. 12 is a sequence of -d5 Fw primer used in Example 1.
SEQ ID NO. 13 is a sequence of -d6 Fw primer used in Example 1.
SEQ ID NO. 14 is a sequence of 6MIEp Rv primer used in Example 1.
SEQ ID NO. 15 is a sequence of 6MIEp-in1 Rv primer used in Example 1.
SEQ ID NO. 16 is a sequence of 6MIEp-in2 Rv primer used in Example 1.
SEQ ID NO. 17 is a sequence of 6MIEp-in3 Rv primer used in Example 1.
SEQ ID NO. 18 is a sequence of 6MIEp-in4 Rv primer used in Example 1.
SEQ ID NO. 19 is a sequence of 6MIEp (-13 to -983; 971 bp based on a transcription initiation point of IE1 contained in MIE of HHV-6B) used in Example 1.
SEQ ID NO. 20 is a sequence of 6MIEp-dl (-13 to -732; 720 bp based on a transcription initiation point of IE1 contained in MIE of HHV-6B) used in Example 1.
SEQ ID NO. 21 is a sequence of 6MIEp-d2(-13 to -552; 540 bp based on a transcription initiation point of IE1 contained in MIE of HHV-6B) used in Example 1.
SEQ ID NO. 22 is a sequence of 6MIEp-d3(-13 to -381; 369 bp based on a transcription initiation point of IE1 contained in MIE of HHV-6B) used in Example 1.
SEQ ID NO. 23 is a sequence of 6MIEp-d4 (-13 to -214; 202 bp based on a transcription initiation point of IE1 contained in MIE of HHV-6B) used in Example 1.
SEQ ID NO. 24 is a sequence of 6MIEp-d5(-13 to -165; 153 bp based on a transcription initiation point of IE1 contained in MIE of HHV-6B) used in Example 1.
SEQ ID NO. 25 is a sequence of 6MIEp-d6(-13 to -102; 90 bp based on a transcription initiation point of IE1 contained in MIE of HHV-6B) used in Example 1.
SEQ ID NO. 26 is a sequence of 6MIEp-in1(+262 to -983; 1245 bp based on a transcription initiation point of IE1 contained in MIE of HHV-6B) used in Example 1.
SEQ ID NO. 27 is a sequence of 6MIEp-dlin1(+262 to -732; 994 bp based on a transcription initiation point of IE1 contained in MIE of HHV-6B) used in Example 1.
SEQ ID NO. 28 is a sequence of 6MIEp-d2in1 (+262 to -552; 814 bp based on a transcription initiation point of IE1 contained in MIE of HHV-6B) used in Example 1.
SEQ ID NO. 29 is a sequence of 6MIEp-d3in1 (+262 to -381; 643 bp based on a transcription initiation point of IE1 contained in MIE of HHV-6B) used in Example 1.
SEQ ID NO. 30 is a sequence of 6MIEp-d4in1 (+262 to -214; 476 bp based on a transcription initiation point of IE1 contained in MIE of HHV-6B) used in Example 1.
SEQ ID NO. 31 is a sequence of 6MIEp-d5in1 (+262 to -165; 427 bp based on a transcription initiation point of IE1 contained in MIE of HHV-6B) used in Example 1.
SEQ ID NO. 32 is a sequence of 6MIEp-d6in1 (+262 to -102; 364 bp based on a transcription initiation point of IE1 contained in MIE of HHV-6B) used in Example 1.
SEQ ID NO. 33 is a sequence of 6MIEp-d2in2 (+1292 to -552; 1844 bp based on a transcription initiation point of IE1 contained in MIE of HHV-6B) used in Example 1.
SEQ ID NO. 34 is a sequence of 6MIEp-d2in3(+1592 to -552; 2144 bp based on a transcription initiation point of IE1 contained in MIE of HHV-6B) used in Example 1.
SEQ ID NO. 35 is a sequence of 6MIEp-d2in4(+1732 to -552; 2284 bp based on a transcription initiation point of IE1 contained in MIE of HHV-6B) used in Example 1.
SEQ ID NO. 36 is a sequence of CMV IE promoter used in Example 1.
SEQ ID NO. 37 is a sequence of U90pA sequence cloned in Example 2.
SEQ ID NO. 38 is a sequence of U100pA sequence cloned in Example 2.
SEQ ID NO. 39 is a sequence of plasmid pBleuScriptSK(-)-6MIE-DsRed2-U90pA constructed in Example 2.
SEQ ID NO. 40 is a sequence of plasmid pBleuScriptSK(-)-6MIE-DsRed2-U100pA constructed in Example 2.
SEQ ID NO. 41 is a sequence of IE1 of HHV-6.

## Claims

1. An enhancer for a promoter, comprising: an intron sequence for a major immediate early gene (MIE) of human herpes virus-6 (HHV-6) (hereinafter, referred to as HHV-6B) or a fragment of the intron sequence.

2. The enhancer for a promoter according to claim 1, wherein the intron sequence includes a sequence (SEQ ID No. 2) of at least -12 to +1292 based on a transcription initiation point of IE1 included in the 6MIE sequence, or a fragment of the sequence.

3. The enhancer for a promoter according to claim 1, wherein the sequence includes a sequence (SEQ ID No. 3) of -12 to +262 based on a transcription initiation point of IE1 included in the 6MIE sequence.

4. The enhancer for a promoter according to claim 1, wherein the sequence is the sequence (SEQ ID No. 3) of -12 to +262 based on a transcription initiation point of IE1 included in the 6MIE sequence.

5. The enhancer for a promoter according to claim 1, wherein the promoter includes a sequence (SEQ ID No. 4) of at least -382 to -983 based on a transcription initiation point of IE1 included in the 6MIE sequence, or a fragment of the sequence.

6. A promoter with improved activity, comprising: an intron sequence for a major immediate early gene (MIE) of human herpes virus-6 (HHV-6) or a fragment of the sequence; and a promoter.

7. The promoter with improved activity according to claim 6, further comprising: a sequence (SEQ ID No. 2) of at least -12 to +1292 based on a transcription initiation point of IE1 included in the 6MIE sequence, or a fragment of the sequence; and an MIE promoter.

8. The promoter with improved activity according to claim 7, wherein the MIE promoter includes a sequence (SEQ ID No. 4) of at least -382 to -983 based on the transcription initiation point of IE1 included in the 6MIE sequence, or a fragment of the sequence.

9. A construct for enhancing expression of a gene in a cell, comprising: an intron sequence for a major immediate early gene (MIE) of human herpes virus-6 (HHV-6) or a fragment of the sequence; and a promoter.

10. The construct according to claim 9, wherein the construct includes: a sequence (SEQ ID No. 2) of at least -12 to +1292 based on a transcription initiation point of IE1 included in the 6MIE sequence or a fragment of the sequence; and an MIE promoter.

11. The construct according to claim 9, wherein the cell is a cell of T-lymphocyte or adherent line.

12. The construct according to claim 9, wherein the cell is selected from the group consisting of Molt-3, Jurkat, MRC-5, MeWo, SupT1, and U373.

13. A method for enhancing expression of a gene in a cell, comprising the steps of:
1) generating a construct including an enhancer for a promoter as described in claim 1 and a promoter, where the gene is arranged so as to be operatively linked to a sequence;
2) introducing the construct into the cell; and
3) culturing the cell under conditions for expressing the gene.

14. Use of a sequence (SEQ ID No. 2) of -12 to +1292 based on a transcription initiation point of IE1 included in the 6MIE sequence, or a fragment of the sequence, as an enhancer for a promoter.


SEQUENCE LISTING

<110>  National Institute of Biomedical Innovation
       The Research Foundation for
       Microbial Diseases of Osaka University

<120>  Enhancer for Promoter, and use thereof

<130>  P/68047.EP01

<140>  EP10813526.0 (PCT/JP2010/005447)
<141>  2010-09-03

<150>  JP2009-205365
<151>  2009-09-04

<160>  41

<170>  PatentIn version 3.3

<210>  1
<211>  1242
<212>  DNA
<213>  Artificial

<220>
<223>  HHV-6B MIE promoter

<400>  1
agtgggtacc gcggttgggg tctttcctac ctaggctaac gagaacccta aaatctgcta      60

acggagcaac cgcagttcca gtttttctca taaaattaaa ggttagtggg taccgcggtt     120

gaaatctttc ctgctaataa ggatgagaac ttcaaaatct cgtaacgcgg caaccgcagt     180

tcctgttttt ctcataaagt taaagatcag cgggtaccgc ggttgaagta tttcctgccc     240

aggcgaatga gaactctaaa agctcgtaac gcggcaaccg agttcctgtt tttcccataa     300

agttaaagat cagcgggtac cgcggttgaa gtatttcctg tccaggcgaa tgagaactct     360

aaaagctcgt aacgcggcaa tcgcagttcc tgtttttctc ataaatttaa agatcagcgg     420

gtaccgcggt tgaagtattt cctgcccagg cgaatgagaa ctctaaaagc tcgtaacgcg     480

gcaaccgcag ttcctgtttt tcttataaag ttaaaaatta gcagatattg cggttgaagt     540

ctttcctgtt catgcgaatt aaaactctaa aaactgctaa cgaagcaacc gagttcctgt     600

ttttctcatt aagttaaagg ttagtgggta ctgtggttgg ggtctttcct acccaggcta     660

acgagaaccc taaaatctgc taacagagca accgcagttc ctgtttttct cataaagtta     720

aaggtcagtg tgtaccgcgg ttacaacatt ttcccctgac taagtcattt atttcgtgag     780

aagcgctaac accaaaacca cattcctgtt tcatgatgtg tagcagatgt ttttaaaaaa     840

aaaacatgac aatttatcag taaagtgttc tttattatcc cgccttcaac cgcaaactcc     900

gtctttctca taaaaaaata caagtcagcc ataagaagaa aacctcaaaa aatccagacc     960

acaaattcct gtttttgagt aagatatgac aaaaccctaa atttttgtaa gcatcagcta    1020

atttccattc catatttgtc taaaaggggt gtatttctac acttgcggtt taacattata    1080

cagcgattgg ctccttcatc ctcgtcattt tcctgtacat cacacccgct atagaattgt 1140

atataagcag aagttacagc cagttcagtg ccacttttct caagaagtgg ctccggagaa 1200

cattctcatc acagagattc tttcttatat cgctgcagtc tg 1242

<210> 2
<211> 1304
<212> DNA
<213> Artificial

<220>
<223> -12 to +1292 from transcription initiation point of HHV-6B MIE

<400> 2
tgccactttt ctcaagaagt ggctccggag aacattctca tcacagagat tctttcttat 60

atcgctgcag tctggtaagt caatttttta actattaata tttaaaattg ctacaaaaat 120

tattttaaat tgaatttaag aatgtttaaa acaatttaat gatattttca tgcgcataat 180

tgtaatttga taagcacgaa agaatttata ctgttcacat ttaatcttac agatttgtga 240

cattgattac atcccatcaa ttattggatt gctggatttt cttcccgccc agaagaaaga 300

ttgattgcac ccgaaaccac cacctggaat caatctccaa agaaacctcc accttgaatc 360

actcttaaaa ggtaagaatt tatattatac atactttaac attcgcatat aggcagaaca 420

gtcaggtatt ttccccaaat ataaactctc cgtacatctc tgtaaagaaa aatacagaca 480

ttacacattc atttcagttt tcggtataat agcctcattt aaagagtcta aagtctctac 540

tgaagaagca taagattgca cttcactctt ctctgtatct ttttctgtgg tccaggaaca 600

tgtaatgaat atcatcatta tacaaaacag taggataatt ccagatgctg tagttaaagc 660

ctgaacccaa cttggagatt tcccttcaat ttttttcctc gattctttgt ttttcgtggg 720

ttggctcgtc acagaatcta aaaacaaacc atccgtgatt tttccattct taaggaatgt 780

atgcaaatat ataatcaata cattaacata aaaaagacat atctttcact aaccggtatg 840

tacagaagta gtggattcag tgcagctggc aagagatgtg cttacatgct tcagcgccac 900

agtcacagct tcgagatcag tagtggtacg tttttggttc gatataattt ccagcgttaa 960

catagttatt gccgcagcta gtattaaaac gatcagacat gccagtatct ttaaacgagt 1020

ctttccagta gctgacactt tttcttcata tcccatcatg atcagtgtta cagattttca 1080

agcagagaat aatctctgca aagaaagagg tggtggtaat ctccttatat agtattatat 1140

ctgctgacag aagatatttt ttcattggct aacagaacag taggcggtgt ctgaatttgc 1200

atcttaatat acaatggagt cagcaaaaga tacaacccct acttctatgt tcattctcgg 1260

aaaaccctct ggaaacaaca tggaatccaa tgaggaacgt atgc 1304

<210> 3
<211> 274
<212> DNA
<213> Artificial

<220>
<223>  -12 to +262 from transcription initiation point of HHV-6B MIE

<400>  3
tgccactttt ctcaagaagt ggctccggag aacattctca tcacagagat tctttcttat      60

atcgctgcag tctggtaagt caatttttta actattaata tttaaaattg ctacaaaaat     120

tattttaaat tgaatttaag aatgtttaaa acaatttaat gatattttca tgcgcataat     180

tgtaatttga taagcacgaa agaatttata ctgttcacat ttaatcttac agatttgtga     240

cattgattac atcccatcaa ttattggatt gctg                                 274


<210>  4
<211>  602
<212>  DNA
<213>  Artificial

<220>
<223>  -382 to -983 from transcription initiation point of HHV-6B MIE

<400>  4
agttaaagat cagcgggtac cgcggttgaa gtatttcctg cccaggcgaa tgagaactct      60

aaaagctcgt aacgcggcaa ccgagttcct gtttttccca taaagttaaa gatcagcggg     120

taccgcggtt gaagtatttc ctgtccaggc gaatgagaac tctaaaagct cgtaacgcgg     180

caatcgcagt tcctgttttt ctcataaatt taaagatcag cgggtaccgc ggttgaagta     240

tttcctgccc aggcgaatga gaactctaaa agctcgtaac gcggcaaccg cagttcctgt     300

ttttcttata aagttaaaaa ttagcagata ttgcggttga agtctttcct gttcatgcga     360

attaaaactc taaaaactgc taacgaagca accgagttcc tgtttttctc attaagttaa     420

aggttagtgg gtactgtggt tggggtcttt cctacccagg ctaacgagaa ccctaaaatc     480

tgctaacaga gcaaccgcag ttcctgtttt tctcataaag ttaaaggtca gtgtgtaccg     540

cggttacaac attttcccct gactaagtca tttatttcgt gagaagcgct aacaccaaaa     600

cc                                                                    602


<210>  5
<211>  148
<212>  DNA
<213>  Artificial

<220>
<223>  -530 to -383 from transcription initiation point of HHV-6B MIE

<400>  5
acccaggcta acgagaaccc taaaatctgc taacagagca accgcagttc ctgtttttct      60

cataaagtta aaggtcagtg tgtaccgcgg ttacaacatt ttcccctgac taagtcattt     120

atttcgtgag aagcgctaac accaaaac                                        148


<210>  6

<211>  625
<212>  DNA
<213>  Artificial

<220>
<223>  -1007 to -383 from transcription initiation point of HHV-6B MIE

<400>  6
gcagttcctg tttttctcat aaagttaaag atcagcgggt accgcggttg aagtatttcc      60

tgcccaggcg aatgagaact ctaaaagctg ctaacgcggc aaccgcagtt cctgtttttc     120

ccataaagtt aaagatcagc gggtaccgcg gttgaagtat ttcctgtcca ggcgaatgag     180

aactctaaaa gctgctaacg cggcaatcgc agttcctgtt tttctcataa atttaaagat     240

cagcgggtac cgcggttgaa gtatttcctg cccaggcgaa tgagaactct aaaagctgct     300

aacgcggcaa ccgcagttcc tgtttttctt ataaagttaa aaattagcag atattgcggt     360

tgaagtcttt cctgttcatg cgaattaaaa ctctaaaaac tgctaacgaa gcaaccgcag     420

ttcctgtttt tctcattaag ttaaaggtta gtgggtactg tggttggggt ctttcctacc     480

caggctaacg agaaccctaa aatctgctaa cagagcaacc gcagttcctg tttttctcat     540

aaagttaaag gtcagtgtgt accgcggtta caacattttc ccctgactaa gtcatttatt     600

tcgtgagaag cgctaacacc aaaac                                           625


<210>  7
<211>  29
<212>  DNA
<213>  Artificial

<220>
<223>  full Fw primer in Example 1

<400>  7
tctctcgaga gttaaagatc agcgggtac                                       29


<210>  8
<211>  34
<212>  DNA
<213>  Artificial

<220>
<223>  -d1 Fw primer in Example 1

<400>  8
agtcggtacc ggcgaatgag aactctaaaa gctc                                 34


<210>  9
<211>  34
<212>  DNA
<213>  Artificial

<220>
<223>  -d2 Fw primer in Example 1

<400>  9
agtcggtacc tactgtggtt ggggtctttc ctac                                 34

<210>  10
<211>  35
<212>  DNA
<213>  Artificial

<220>
<223>  -d3 Fw primer in Example 1

<400>  10
agtcggtacc acattcctgt ttcatgatgt gtagc                                35


<210>  11
<211>  34
<212>  DNA
<213>  Artificial

<220>
<223>  -d4 Fw primer in Example 1

<400>  11
agtcggtacc tcctgttttt gagtaagata tgac                                 34


<210>  12
<211>  35
<212>  DNA
<213>  Artificial

<220>
<223>  -d5 Fw primer in Example 1

<400>  12
agtcggtacc agctaatttc cattccatat ttgtc                                35


<210>  13
<211>  35
<212>  DNA
<213>  Artificial

<220>
<223>  -d6 Fw primer in Example 1

<400>  13
agtcggtacc tacagcgatt ggctccttca tcctc                                35


<210>  14
<211>  34
<212>  DNA
<213>  Artificial

<220>
<223>  6MIEp Rv primer in Example 1

<400>  14
agtcctcgag cactgaactg gctgtaactt ctgc                                 34


<210>  15
<211>  29
<212>  DNA

<213>  Artificial

<220>
<223>  6MIEp-in1 Rv primer in Example 1

<400>  15
tctaagcttc agcaatccaa taattgatg                                          29


<210>  16
<211>  29
<212>  DNA
<213>  Artificial

<220>
<223>  6MIEp-in2 Rv primer in Example 1

<400>  16
cataagcttg catacgttcc tcattggat                                          29


<210>  17
<211>  29
<212>  DNA
<213>  Artificial

<220>
<223>  6MIEp-in3 Rv primer in Example 1

<400>  17
cataagcttc caaagttttg aattcttca                                          29


<210>  18
<211>  29
<212>  DNA
<213>  Artificial

<220>
<223>  6MIEp-in4 Rv primer in Example 1

<400>  18
cataagcttt ttggatgcaa gtgccaacg                                          29


<210>  19
<211>  971
<212>  DNA
<213>  Artificial

<220>
<223>  6MIEp sequence in Example 1

<400>  19
agttaaagat cagcgggtac cgcggttgaa gtatttcctg cccaggcgaa tgagaactct      60

aaaagctcgt aacgcggcaa ccgagttcct gtttttccca taaagttaaa gatcagcggg     120

taccgcggtt gaagtatttc ctgtccaggc gaatgagaac tctaaaagct cgtaacgcgg     180

caatcgcagt tcctgttttt ctcataaatt taaagatcag cgggtaccgc ggttgaagta     240

tttcctgccc aggcgaatga gaactctaaa agctcgtaac gcggcaaccg cagttcctgt     300

ttttcttata aagttaaaaa ttagcagata ttgcggttga agtctttcct gttcatgcga     360

attaaaactc taaaaactgc taacgaagca accgagttcc tgtttttctc attaagttaa 420

aggttagtgg gtactgtggt tggggtcttt cctacccagg ctaacgagaa ccctaaaatc 480

tgctaacaga gcaaccgcag ttcctgtttt tctcataaag ttaaaggtca gtgtgtaccg 540

cggttacaac attttcccct gactaagtca tttatttcgt gagaagcgct aacaccaaaa 600

ccacattcct gtttcatgat gtgtagcaga tgtttttaaa aaaaaaacat gacaatttat 660

cagtaaagtg ttctttatta tcccgccttc aaccgcaaac tccgtctttc tcataaaaaa 720

atacaagtca gccataagaa gaaaacctca aaaaatccag accacaaatt cctgtttttg 780

agtaagatat gacaaaaccc taaatttttg taagcatcag ctaatttcca ttccatattt 840

gtctaaaagg ggtgtatttc tacacttgcg gtttaacatt atacagcgat tggctccttc 900

atcctcgtca ttttcctgta catcacaccc gctatagaat tgtatataag cagaagttac 960

agccagttca g 971

<210> 20
<211> 720
<212> DNA
<213> Artificial

<220>
<223> 6MIEp-d1 sequence in Example 1

<400> 20
ggcgaatgag aactctaaaa gctcgtaacg cggcaaccgc agttcctgtt tttcttataa 60

agttaaaaat tagcagatat tgcggttgaa gtctttcctg ttcatgcgaa ttaaaactct 120

aaaaactgct aacgaagcaa ccgagttcct gtttttctca ttaagttaaa ggttagtggg 180

tactgtggtt ggggtctttc ctacccaggc taacgagaac cctaaaatct gctaacagag 240

caaccgcagt tcctgttttt ctcataaagt taaaggtcag tgtgtaccgc ggttacaaca 300

ttttcccctg actaagtcat ttatttcgtg agaagcgcta acaccaaaac cacattcctg 360

tttcatgatg tgtagcagat gtttttaaaa aaaaaacatg acaatttatc agtaaagtgt 420

tctttattat cccgccttca accgcaaact ccgtctttct cataaaaaaa tacaagtcag 480

ccataagaag aaaacctcaa aaaatccaga ccacaaattc ctgtttttga gtaagatatg 540

acaaaaccct aaatttttgt aagcatcagc taatttccat tccatatttg tctaaaaggg 600

gtgtatttct acacttgcgg tttaacatta tacagcgatt ggctccttca tcctcgtcat 660

tttcctgtac atcacacccg ctatagaatt gtatataagc agaagttaca gccagttcag 720

<210> 21
<211> 540
<212> DNA
<213> Artificial

<220>
<223> 6MIEp-d2 sequence in Example 1

<400>  21
tactgtggtt ggggtctttc ctacccaggc taacgagaac cctaaaatct gctaacagag      60

caaccgcagt tcctgttttt ctcataaagt taaaggtcag tgtgtaccgc ggttacaaca     120

ttttcccctg actaagtcat ttatttcgtg agaagcgcta acaccaaaac cacattcctg     180

tttcatgatg tgtagcagat gtttttaaaa aaaaaacatg acaatttatc agtaaagtgt     240

tctttattat cccgccttca accgcaaact ccgtctttct cataaaaaaa tacaagtcag     300

ccataagaag aaaacctcaa aaaatccaga ccacaaattc ctgtttttga gtaagatatg     360

acaaaaccct aaatttttgt aagcatcagc taatttccat tccatatttg tctaaaaggg     420

gtgtatttct acacttgcgg tttaacatta tacagcgatt ggctccttca tcctcgtcat     480

tttcctgtac atcacacccg ctatagaatt gtatataagc agaagttaca gccagttcag     540


<210>  22
<211>  369
<212>  DNA
<213>  Artificial

<220>
<223>  6MIEp-d3 sequence in Example 1

<400>  22
acattcctgt ttcatgatgt gtagcagatg tttttaaaaa aaaaacatga caatttatca      60

gtaaagtgtt ctttattatc ccgccttcaa ccgcaaactc cgtctttctc ataaaaaaat     120

acaagtcagc cataagaaga aaacctcaaa aaatccagac cacaaattcc tgtttttgag     180

taagatatga caaaacccta aatttttgta agcatcagct aatttccatt ccatatttgt     240

ctaaaagggg tgtatttcta cacttgcggt ttaacattat acagcgattg gctccttcat     300

cctcgtcatt ttcctgtaca tcacacccgc tatagaattg tatataagca gaagttacag     360

ccagttcag                                                             369


<210>  23
<211>  202
<212>  DNA
<213>  Artificial

<220>
<223>  6MIEp-d4 sequence in Example 1

<400>  23
tcctgttttt gagtaagata tgacaaaacc ctaaattttt gtaagcatca gctaatttcc      60

attccatatt tgtctaaaag gggtgtattt ctacacttgc ggtttaacat tatacagcga     120

ttggctcctt catcctcgtc attttcctgt acatcacacc cgctatagaa ttgtatataa     180

gcagaagtta cagccagttc ag                                              202


<210>  24
<211>  153

<212>  DNA
<213>  Artificial

<220>
<223>  6MIEp-d5 sequence in Example 1

<400>  24
agctaatttc cattccatat ttgtctaaaa ggggtgtatt tctacacttg cggtttaaca      60

ttatacagcg attggctcct tcatcctcgt cattttcctg tacatcacac ccgctataga     120

attgtatata agcagaagtt acagccagtt cag                                  153


<210>  25
<211>  90
<212>  DNA
<213>  Artificial

<220>
<223>  6MIEp-d6 sequence in Example 1

<400>  25
tacagcgatt ggctccttca tcctcgtcat tttcctgtac atcacacccg ctatagaatt      60

gtatataagc agaagttaca gccagttcag                                       90


<210>  26
<211>  1245
<212>  DNA
<213>  Artificial

<220>
<223>  6MIEp-in1 sequence in Example 1

<400>  26
agttaaagat cagcgggtac cgcggttgaa gtatttcctg cccaggcgaa tgagaactct      60

aaaagctcgt aacgcggcaa ccgagttcct gtttttccca taaagttaaa gatcagcggg     120

taccgcggtt gaagtatttc ctgtccaggc gaatgagaac tctaaaagct cgtaacgcgg     180

caatcgcagt tcctgttttt ctcataaatt taaagatcag cgggtaccgc ggttgaagta     240

tttcctgccc aggcgaatga gaactctaaa agctcgtaac gcggcaaccg cagttcctgt     300

ttttcttata aagttaaaaa ttagcagata ttgcggttga agtctttcct gttcatgcga     360

attaaaactc taaaaactgc taacgaagca accgagttcc tgtttttctc attaagttaa     420

aggttagtgg gtactgtggt tggggtcttt cctacccagg ctaacgagaa ccctaaaatc     480

tgctaacaga gcaaccgcag ttcctgtttt tctcataaag ttaaaggtca gtgtgtaccg     540

cggttacaac attttcccct gactaagtca tttatttcgt gagaagcgct aacaccaaaa     600

ccacattcct gtttcatgat gtgtagcaga tgtttttaaa aaaaaaacat gacaatttat     660

cagtaaagtg ttctttatta tcccgccttc aaccgcaaac tccgtctttc tcataaaaaa     720

atacaagtca gccataagaa gaaaacctca aaaaatccag accacaaatt cctgtttttg     780

agtaagatat gacaaaaccc taaatttttg taagcatcag ctaatttcca ttccatattt     840

gtctaaaagg ggtgtatttc tacacttgcg gtttaacatt atacagcgat tggctccttc 900

atcctcgtca ttttcctgta catcacaccc gctatagaat tgtatataag cagaagttac 960

agccagttca gtgccacttt tctcaagaag tggctccgga gaacattctc atcacagaga 1020

ttctttctta tatcgctgca gtctggtaag tcaatttttt aactattaat atttaaaatt 1080

gctacaaaaa ttattttaaa ttgaatttaa gaatgtttaa aacaatttaa tgatattttc 1140

atgcgcataa ttgtaatttg ataagcacga aagaatttat actgttcaca tttaatctta 1200

cagatttgtg acattgatta catcccatca attattggat tgctg 1245

<210> 27
<211> 994
<212> DNA
<213> Artificial

<220>
<223> 6MIEp-dlin1 sequence in Example 1

<400> 27
ggcgaatgag aactctaaaa gctcgtaacg cggcaaccgc agttcctgtt tttcttataa 60

agttaaaaat tagcagatat tgcggttgaa gtctttcctg ttcatgcgaa ttaaaactct 120

aaaaactgct aacgaagcaa ccgagttcct gtttttctca ttaagttaaa ggttagtggg 180

tactgtggtt ggggtctttc ctacccaggc taacgagaac cctaaaatct gctaacagag 240

caaccgcagt tcctgttttt ctcataaagt taaaggtcag tgtgtaccgc ggttacaaca 300

ttttcccctg actaagtcat ttatttcgtg agaagcgcta acaccaaaac cacattcctg 360

tttcatgatg tgtagcagat gtttttaaaa aaaaaacatg acaatttatc agtaaagtgt 420

tctttattat cccgccttca accgcaaact ccgtctttct cataaaaaaa tacaagtcag 480

ccataagaag aaaacctcaa aaaatccaga ccacaaattc ctgtttttga gtaagatatg 540

acaaaaccct aaatttttgt aagcatcagc taatttccat tccatatttg tctaaaaggg 600

gtgtatttct acacttgcgg tttaacatta tacagcgatt ggctccttca tcctcgtcat 660

tttcctgtac atcacacccg ctatagaatt gtatataagc agaagttaca gccagttcag 720

tgccactttt ctcaagaagt ggctccggag aacattctca tcacagagat tctttcttat 780

atcgctgcag tctggtaagt caatttttta actattaata tttaaaattg ctacaaaaat 840

tattttaaat tgaatttaag aatgtttaaa acaatttaat gatattttca tgcgcataat 900

tgtaatttga taagcacgaa agaatttata ctgttcacat ttaatcttac agatttgtga 960

cattgattac atcccatcaa ttattggatt gctg 994

<210> 28
<211> 814
<212> DNA
<213> Artificial

<220>

<223>  6MIEp-d2in1 sequence in Example 1

<400>  28
tactgtggtt ggggtctttc ctacccaggc taacgagaac cctaaaatct gctaacagag      60

caaccgcagt tcctgttttt ctcataaagt taaaggtcag tgtgtaccgc ggttacaaca     120

ttttcccctg actaagtcat ttatttcgtg agaagcgcta acaccaaaac cacattcctg     180

tttcatgatg tgtagcagat gtttttaaaa aaaaaacatg acaatttatc agtaaagtgt     240

tctttattat cccgccttca accgcaaact ccgtctttct cataaaaaaa tacaagtcag     300

ccataagaag aaaacctcaa aaaatccaga ccacaaattc ctgtttttga gtaagatatg     360

acaaaaccct aaatttttgt aagcatcagc taatttccat tccatatttg tctaaaaggg     420

gtgtatttct acacttgcgg tttaacatta tacagcgatt ggctccttca tcctcgtcat     480

tttcctgtac atcacacccg ctatagaatt gtatataagc agaagttaca gccagttcag     540

tgccactttt ctcaagaagt ggctccggag aacattctca tcacagagat tctttcttat     600

atcgctgcag tctggtaagt caatttttta actattaata tttaaaattg ctacaaaaat     660

tattttaaat tgaatttaag aatgtttaaa acaatttaat gatattttca tgcgcataat     720

tgtaatttga taagcacgaa agaatttata ctgttcacat ttaatcttac agatttgtga     780

cattgattac atcccatcaa ttattggatt gctg                                 814


<210>  29
<211>  643
<212>  DNA
<213>  Artificial

<220>
<223>  6MIEp-d3in1 sequence in Example 1

<400>  29
acattcctgt ttcatgatgt gtagcagatg tttttaaaaa aaaaacatga caatttatca      60

gtaaagtgtt ctttattatc ccgccttcaa ccgcaaactc cgtctttctc ataaaaaaat     120

acaagtcagc cataagaaga aaacctcaaa aaatccagac cacaaattcc tgtttttgag     180

taagatatga caaaacccta aatttttgta agcatcagct aatttccatt ccatatttgt     240

ctaaaagggg tgtatttcta cacttgcggt ttaacattat acagcgattg gctccttcat     300

cctcgtcatt ttcctgtaca tcacacccgc tatagaattg tatataagca gaagttacag     360

ccagttcagt gccacttttc tcaagaagtg gctccggaga acattctcat cacagagatt     420

ctttcttata tcgctgcagt ctggtaagtc aattttttaa ctattaatat ttaaaattgc     480

tacaaaaatt attttaaatt gaatttaaga atgtttaaaa caatttaatg atattttcat     540

gcgcataatt gtaatttgat aagcacgaaa gaatttatac tgttcacatt taatcttaca     600

gatttgtgac attgattaca tcccatcaat tattggattg ctg                       643


<210>  30

<211>  476
<212>  DNA
<213>  Artificial

<220>
<223>  6MIEp-d4in1 sequence in Example 1

<400>  30
tcctgttttt gagtaagata tgacaaaacc ctaaattttt gtaagcatca gctaatttcc      60

attccatatt tgtctaaaag gggtgtattt ctacacttgc ggtttaacat tatacagcga     120

ttggctcctt catcctcgtc attttcctgt acatcacacc cgctatagaa ttgtatataa     180

gcagaagtta cagccagttc agtgccactt ttctcaagaa gtggctccgg agaacattct     240

catcacagag attctttctt atatcgctgc agtctggtaa gtcaattttt taactattaa     300

tatttaaaat tgctacaaaa attattttaa attgaattta agaatgttta aaacaattta     360

atgatatttt catgcgcata attgtaattt gataagcacg aaagaattta tactgttcac     420

atttaatctt acagatttgt gacattgatt acatcccatc aattattgga ttgctg         476


<210>  31
<211>  427
<212>  DNA
<213>  Artificial

<220>
<223>  6MIEp-d5in1 sequence in Example 1

<400>  31
agctaatttc cattccatat ttgtctaaaa ggggtgtatt tctacacttg cggtttaaca      60

ttatacagcg attggctcct tcatcctcgt cattttcctg tacatcacac ccgctataga     120

attgtatata agcagaagtt acagccagtt cagtgccact tttctcaaga agtggctccg     180

gagaacattc tcatcacaga gattctttct tatatcgctg cagtctggta agtcaatttt     240

ttaactatta atatttaaaa ttgctacaaa aattatttta aattgaattt aagaatgttt     300

aaaacaattt aatgatattt tcatgcgcat aattgtaatt tgataagcac gaaagaattt     360

atactgttca catttaatct tacagatttg tgacattgat tacatcccat caattattgg     420

attgctg                                                               427


<210>  32
<211>  364
<212>  DNA
<213>  Artificial

<220>
<223>  6MIEp-d6in1 sequence in Example 1

<400>  32
tacagcgatt ggctccttca tcctcgtcat tttcctgtac atcacacccg ctatagaatt      60

gtatataagc agaagttaca gccagttcag tgccactttt ctcaagaagt ggctccggag     120

aacattctca tcacagagat tctttcttat atcgctgcag tctggtaagt caatttttta     180

actattaata tttaaaattg ctacaaaaat tattttaaat tgaatttaag aatgtttaaa 240

acaatttaat gatattttca tgcgcataat tgtaatttga taagcacgaa agaatttata 300

ctgttcacat ttaatcttac agatttgtga cattgattac atcccatcaa ttattggatt 360

gctg 364

<210> 33
<211> 1844
<212> DNA
<213> Artificial

<220>
<223> 6MIEp-d2in2 sequence in Example 1

<400> 33
tactgtggtt ggggtctttc ctacccaggc taacgagaac cctaaaatct gctaacagag 60

caaccgcagt tcctgttttt ctcataaagt taaaggtcag tgtgtaccgc ggttacaaca 120

ttttcccctg actaagtcat ttatttcgtg agaagcgcta acaccaaaac cacattcctg 180

tttcatgatg tgtagcagat gtttttaaaa aaaaaacatg acaatttatc agtaaagtgt 240

tctttattat cccgccttca accgcaaact ccgtctttct cataaaaaaa tacaagtcag 300

ccataagaag aaaacctcaa aaaatccaga ccacaaattc ctgtttttga gtaagatatg 360

acaaaaccct aaatttttgt aagcatcagc taatttccat tccatatttg tctaaaaggg 420

gtgtatttct acacttgcgg tttaacatta tacagcgatt ggctccttca tcctcgtcat 480

tttcctgtac atcacacccg ctatagaatt gtatataagc agaagttaca gccagttcag 540

tgccactttt ctcaagaagt ggctccggag aacattctca tcacagagat tctttcttat 600

atcgctgcag tctggtaagt caatttttta actattaata tttaaaattg ctacaaaaat 660

tattttaaat tgaatttaag aatgtttaaa acaatttaat gatattttca tgcgcataat 720

tgtaatttga taagcacgaa agaatttata ctgttcacat ttaatcttac agatttgtga 780

cattgattac atcccatcaa ttattggatt gctggatttt cttcccgccc agaagaaaga 840

ttgattgcac ccgaaaccac cacctggaat caatctccaa agaaacctcc accttgaatc 900

actcttaaaa ggtaagaatt tatattatac atactttaac attcgcatat aggcagaaca 960

gtcaggtatt ttccccaaat ataaactctc cgtacatctc tgtaaagaaa aatacagaca 1020

ttacacattc atttcagttt tcggtataat agcctcattt aaagagtcta aagtctctac 1080

tgaagaagca taagattgca cttcactctt ctctgtatct ttttctgtgg tccaggaaca 1140

tgtaatgaat atcatcatta tacaaaacag taggataatt ccagatgctg tagttaaagc 1200

ctgaacccaa cttggagatt tcccttcaat ttttttcctc gattctttgt ttttcgtggg 1260

ttggctcgtc acagaatcta aaaacaaacc atccgtgatt tttccattct taaggaatgt 1320

atgcaaatat ataatcaata cattaacata aaaaagacat atctttcact aaccggtatg 1380

tacagaagta gtggattcag tgcagctggc aagagatgtg cttacatgct tcagcgccac 1440

agtcacagct tcgagatcag tagtggtacg tttttggttc gatataattt ccagcgttaa 1500

catagttatt gccgcagcta gtattaaaac gatcagacat gccagtatct ttaaacgagt 1560

ctttccagta gctgacactt tttcttcata tcccatcatg atcagtgtta cagattttca 1620

agcagagaat aatctctgca aagaaagagg tggtggtaat ctccttatat agtattatat 1680

ctgctgacag aagatatttt ttcattggct aacagaacag taggcggtgt ctgaatttgc 1740

atcttaatat acaatggagt cagcaaaaga tacaacccct acttctatgt tcattctcgg 1800

aaaaccctct ggaaacaaca tggaatccaa tgaggaacgt atgc 1844

<210> 34
<211> 2144
<212> DNA
<213> Artificial

<220>
<223> 6MIEp-d2in3 sequence in Example 1

<400> 34
tactgtggtt ggggtctttc ctacccaggc taacgagaac cctaaaatct gctaacagag 60

caaccgcagt tcctgttttt ctcataaagt taaaggtcag tgtgtaccgc ggttacaaca 120

ttttcccctg actaagtcat ttatttcgtg agaagcgcta acaccaaaac cacattcctg 180

tttcatgatg tgtagcagat gtttttaaaa aaaaaacatg acaatttatc agtaaagtgt 240

tctttattat cccgccttca accgcaaact ccgtctttct cataaaaaaa tacaagtcag 300

ccataagaag aaaacctcaa aaaatccaga ccacaaattc ctgtttttga gtaagatatg 360

acaaaaccct aaatttttgt aagcatcagc taatttccat tccatatttg tctaaaaggg 420

gtgtatttct acacttgcgg tttaacatta tacagcgatt ggctccttca tcctcgtcat 480

tttcctgtac atcacacccg ctatagaatt gtatataagc agaagttaca gccagttcag 540

tgccactttt ctcaagaagt ggctccggag aacattctca tcacagagat tctttcttat 600

atcgctgcag tctggtaagt caatttttta actattaata tttaaaattg ctacaaaaat 660

tattttaaat tgaatttaag aatgtttaaa acaatttaat gatattttca tgcgcataat 720

tgtaatttga taagcacgaa agaatttata ctgttcacat ttaatcttac agatttgtga 780

cattgattac atcccatcaa ttattggatt gctggatttt cttcccgccc agaagaaaga 840

ttgattgcac ccgaaaccac cacctggaat caatctccaa agaaacctcc accttgaatc 900

actcttaaaa ggtaagaatt tatattatac atactttaac attcgcatat aggcagaaca 960

gtcaggtatt ttccccaaat ataaactctc cgtacatctc tgtaaagaaa aatacagaca 1020

ttacacattc atttcagttt tcggtataat agcctcattt aaagagtcta aagtctctac 1080

tgaagaagca taagattgca cttcactctt ctctgtatct ttttctgtgg tccaggaaca 1140

tgtaatgaat atcatcatta tacaaaacag taggataatt ccagatgctg tagttaaagc 1200

ctgaacccaa cttggagatt tcccttcaat ttttttcctc gattctttgt ttttcgtggg 1260

ttggctcgtc acagaatcta aaaacaaacc atccgtgatt tttccattct taaggaatgt 1320

atgcaaatat ataatcaata cattaacata aaaaagacat atctttcact aaccggtatg 1380

tacagaagta gtggattcag tgcagctggc aagagatgtg cttacatgct tcagcgccac 1440

agtcacagct tcgagatcag tagtggtacg tttttggttc gatataattt ccagcgttaa 1500

catagttatt gccgcagcta gtattaaaac gatcagacat gccagtatct ttaaacgagt 1560

ctttccagta gctgacactt tttcttcata tcccatcatg atcagtgtta cagattttca 1620

agcagagaat aatctctgca aagaaagagg tggtggtaat ctccttatat agtattatat 1680

ctgctgacag aagatatttt ttcattggct aacagaacag taggcggtgt ctgaatttgc 1740

atcttaatat acaatggagt cagcaaaaga tacaacccct acttctatgt tcattctcgg 1800

aaaaccctct ggaaacaaca tggaatccaa tgaggaacgt atgcaaaagt aagtatgatt 1860

agtccttatt agatctatct tatttaaaac tatatgggag tttttctaag tactaagaaa 1920

tatccatact tacagctacc atcccgatcc agtggtggaa gaatccatca aagaaatatt 1980

ggaagagagt ctcaagtgtg atgtgtcctt tgaaagcctg ctttttccag aactggaagc 2040

ctttgatctt ttcatcccag agtcttccaa cgacatcgct tccaagaatg tgtcctattc 2100

cagtaatgtg gaagaaggag catctgaaga attcaaaact ttgg 2144

<210> 35
<211> 2284
<212> DNA
<213> Artificial

<220>
<223> 6MIEp-d2in4 sequence in Example 1

<400> 35
tactgtggtt ggggtctttc ctacccaggc taacgagaac cctaaaatct gctaacagag 60

caaccgcagt tcctgttttt ctcataaagt taaaggtcag tgtgtaccgc ggttacaaca 120

ttttcccctg actaagtcat ttatttcgtg agaagcgcta acaccaaaac cacattcctg 180

tttcatgatg tgtagcagat gtttttaaaa aaaaaacatg acaatttatc agtaaagtgt 240

tctttattat cccgccttca accgcaaact ccgtctttct cataaaaaaa tacaagtcag 300

ccataagaag aaaacctcaa aaaatccaga ccacaaattc ctgtttttga gtaagatatg 360

acaaaaccct aaatttttgt aagcatcagc taatttccat tccatatttg tctaaaaggg 420

gtgtatttct acacttgcgg tttaacatta tacagcgatt ggctccttca tcctcgtcat 480

tttcctgtac atcacacccg ctatagaatt gtatataagc agaagttaca gccagttcag 540

tgccactttt ctcaagaagt ggctccggag aacattctca tcacagagat tctttcttat 600

atcgctgcag tctggtaagt caatttttta actattaata tttaaaattg ctacaaaaat 660

tattttaaat tgaatttaag aatgtttaaa acaatttaat gatattttca tgcgcataat 720

tgtaatttga taagcacgaa agaatttata ctgttcacat ttaatcttac agatttgtga 780

cattgattac atcccatcaa ttattggatt gctggatttt cttcccgccc agaagaaaga 840

ttgattgcac ccgaaaccac cacctggaat caatctccaa agaaacctcc accttgaatc 900

actcttaaaa ggtaagaatt tatattatac atactttaac attcgcatat aggcagaaca 960

gtcaggtatt ttccccaaat ataaactctc cgtacatctc tgtaaagaaa aatacagaca 1020

ttacacattc atttcagttt tcggtataat agcctcattt aaagagtcta aagtctctac 1080

tgaagaagca taagattgca cttcactctt ctctgtatct ttttctgtgg tccaggaaca 1140

tgtaatgaat atcatcatta tacaaaacag taggataatt ccagatgctg tagttaaagc 1200

ctgaacccaa cttggagatt tcccttcaat ttttttcctc gattctttgt ttttcgtggg 1260

ttggctcgtc acagaatcta aaaacaaacc atccgtgatt tttccattct taaggaatgt 1320

atgcaaatat ataatcaata cattaacata aaaaagacat atctttcact aaccggtatg 1380

tacagaagta gtggattcag tgcagctggc aagagatgtg cttacatgct tcagcgccac 1440

agtcacagct tcgagatcag tagtggtacg tttttggttc gatataattt ccagcgttaa 1500

catagttatt gccgcagcta gtattaaaac gatcagacat gccagtatct ttaaacgagt 1560

ctttccagta gctgacactt tttcttcata tcccatcatg atcagtgtta cagattttca 1620

agcagagaat aatctctgca aagaaagagg tggtggtaat ctccttatat agtattatat 1680

ctgctgacag aagatatttt ttcattggct aacagaacag taggcggtgt ctgaatttgc 1740

atcttaatat acaatggagt cagcaaaaga tacaacccct acttctatgt tcattctcgg 1800

aaaaccctct ggaaacaaca tggaatccaa tgaggaacgt atgcaaaagt aagtatgatt 1860

agtccttatt agatctatct tatttaaaac tatatgggag tttttctaag tactaagaaa 1920

tatccatact tacagctacc atcccgatcc agtggtggaa gaatccatca aagaaatatt 1980

ggaagagagt ctcaagtgtg atgtgtcctt tgaaagcctg ctttttccag aactggaagc 2040

ctttgatctt ttcatcccag agtcttccaa cgacatcgct tccaagaatg tgtcctattc 2100

cagtaatgtg gaagaaggag catctgaaga attcaaaact ttggttggtg agtacatgaa 2160

ttatatatta tatgtcccaa gatagaaaac aagaaaaaaa catagtttta agcatgtaac 2220

atatagcata cttagaatat cttcttttac atcctagctc agtccgttgg cacttgcatc 2280

caaa 2284

<210> 36
<211> 655
<212> DNA
<213> Artificial

<220>
<223> CMVIE promoter

<400>  36
cgatgtacgg gccagatata cgcgttgaca ttgattattg actagttatt aatagtaatc      60

aattacgggg tcattagttc atagcccata tatggagttc cgcgttacat aacttacggt     120

aaatggcccg cctggctgac cgcccaacga cccccgccca ttgacgtcaa taatgacgta     180

tgttcccata gtaacgccaa tagggacttt ccattgacgt caatgggtgg agtatttacg     240

gtaaactgcc cacttggcag tacatcaagt gtatcatatg ccaagtacgc cccctattga     300

cgtcaatgac ggtaaatggc ccgcctggca ttatgcccag tacatgacct tatgggactt     360

tcctacttgg cagtacatct acgtattagt catcgctatt accatggtga tgcggttttg     420

gcagtacatc aatgggcgtg gatagcggtt tgactcacgg ggatttccaa gtctccaccc     480

cattgacgtc aatgggagtt tgttttggca ccaaaatcaa cgggactttc caaaatgtcg     540

taacaactcc gccccattga cgcaaatggg cggtaggcgt gtacggtggg aggtctatat     600

aagcagagct ctctggctaa ctagagaacc cactgcttac tggcttatcg aaatt          655


<210>  37
<211>  206
<212>  DNA
<213>  Artificial

<220>
<223>  U90pA sequence in Example 2


<220>
<221>  polyA_signal
<222>  (101)..(106)

<400>  37
ttagaaatta cataagcaaa tgtaactttt tctattatgt aaaacctcag caaacatgtg      60

atttctcaaa ggaatttatt ttcaatatca ccttacaaat aataaaaagt catacagaca     120

ttgctttctc tttttaattc caagtcatgt acataaactg acttataagt catcatacaa     180

tatttccata agttaattcc agcttt                                          206


<210>  38
<211>  154
<212>  DNA
<213>  Artificial

<220>
<223>  U100pA sequence in Example 2


<220>
<221>  polyA_signal
<222>  (49)..(54)

<400>  38
ccctcagacc ctacgttgcc ctcaccttat ggcaacggac attattaaaa taaaaaaatt      60

actgaaagag agtcagaaat tgtgtcacat gttattttat taaatcttac tgaactttgt     120

cctttgtcct tacaaacctt ccctatcaca atac 154

<210> 39
<211> 5066
<212> DNA
<213> Artificial

<220>
<223> pBleuScriptSK(-)-6MIE-DsRed2-U90pA sequence in Example 2

<400> 39
cacctgacgc gccctgtagc ggcgcattaa gcgcggcggg tgtggtggtt acgcgcagcg 60

tgaccgctac acttgccagc gccctagcgc ccgctccttt cgctttcttc ccttcctttc 120

tcgccacgtt cgccggcttt ccccgtcaag ctctaaatcg ggggctccct ttagggttcc 180

gatttagtgc tttacggcac ctcgacccca aaaaacttga ttagggtgat ggttcacgta 240

gtgggccatc gccctgatag acggtttttc gccctttgac gttggagtcc acgttcttta 300

atagtggact cttgttccaa actggaacaa cactcaaccc tatctcggtc tattcttttg 360

atttataagg gattttgccg atttcggcct attggttaaa aaatgagctg atttaacaaa 420

aatttaacgc gaattttaac aaaatattaa cgcttacaat ttccattcgc cattcaggct 480

gcgcaactgt tgggaagggc gatcggtgcg ggcctcttcg ctattacgcc agctggcgaa 540

agggggatgt gctgcaaggc gattaagttg ggtaacgcca gggttttccc agtcacgacg 600

ttgtaaaacg acggccagtg aattgtaata cgactcacta tagggcgaat tgggtaccgg 660

gccccccctc gaggtcgaga gttaaagatc agcgggtacc gcggttgaag tatttcctgc 720

ccaggcgaat gagaactcta aaagctcgta acgcggcaac cgagttcctg tttttcccat 780

aaagttaaag atcagcgggt accgcggttg aagtatttcc tgtccaggcg aatgagaact 840

ctaaaagctc gtaacgcggc aatcgcagtt cctgtttttc tcataaattt aaagatcagc 900

gggtaccgcg gttgaagtat ttcctgccca ggcgaatgag aactctaaaa gctcgtaacg 960

cggcaaccgc agttcctgtt tttcttataa agttaaaaat tagcagatat tgcggttgaa 1020

gtctttcctg ttcatgcgaa ttaaaactct aaaaactgct aacgaagcaa ccgagttcct 1080

gtttttctca ttaagttaaa ggttagtggg tactgtggtt ggggtctttc ctacccaggc 1140

taacgagaac cctaaaatct gctaacagag caaccgcagt tcctgttttt ctcataaagt 1200

taaaggtcag tgtgtaccgc ggttacaaca ttttcccctg actaagtcat ttatttcgtg 1260

agaagcgcta acaccaaaac cacattcctg tttcatgatg tgtagcagat gtttttaaaa 1320

aaaaaacatg acaatttatc agtaaagtgt tctttattat cccgccttca accgcaaact 1380

ccgtctttct cataaaaaaa tacaagtcag ccataagaag aaaacctcaa aaaatccaga 1440

ccacaaattc ctgtttttga gtaagatatg acaaaaccct aaatttttgt aagcatcagc 1500

taatttccat tccatatttg tctaaaaggg gtgtatttct acacttgcgg tttaacatta 1560

tacagcgatt ggctccttca tcctcgtcat tttcctgtac atcacacccg ctatagaatt 1620

gtatataagc agaagttaca gccagttcag tgccactttt ctcaagaagt ggctccggag 1680

aacattctca tcacagagat tctttcttat atcgctgcag tctggtaagt caatttttta 1740

actattaata tttaaaattg ctacaaaaat tattttaaat tgaatttaag aatgtttaaa 1800

acaatttaat gatattttca tgcgcataat tgtaatttga taagcacgaa agaatttata 1860

ctgttcacat ttaatcttac agatttgtga cattgattac atcccatcaa ttattggatt 1920

gctggtcgac ggtatcgata agcttgatat cgaattccgc caccatggcc tcctccgaga 1980

acgtcatcac cgagttcatg cgcttcaagg tgcgcatgga gggcaccgtg aacggccacg 2040

agttcgagat cgagggcgag ggcgagggcc gcccctacga gggccacaac accgtgaagc 2100

tgaaggtgac caagggcggc cccctgccct tcgcctggga catcctgtcc ccccagttcc 2160

agtacggctc caaggtgtac gtgaagcacc ccgccgacat ccccgactac aagaagctgt 2220

ccttccccga gggcttcaag tgggagcgcg tgatgaactt cgaggacggc ggcgtggcga 2280

ccgtgaccca ggactcctcc ctgcaggacg gctgcttcat ctacaaggtg aagttcatcg 2340

gcgtgaactt cccctccgac ggccccgtga tgcagaagaa gaccatgggc tgggaggcct 2400

ccaccgagcg cctgtacccc cgcgacggcg tgctgaaggg cgagacccac aaggccctga 2460

agctgaagga cggcggccac tacctggtgg agttcaagtc catctacatg gccaagaagc 2520

ccgtgcagct gcccggctac tactacgtgg acgccaagct ggacatcacc tcccacaacg 2580

aggactacac catcgtggag cagtacgagc gcaccgaggg ccgccaccac ctgttcctgt 2640

agtctagagc ggccgcttag aaattacata agcaaatgta actttttcta ttatgtaaaa 2700

cctcagcaaa catgtgattt ctcaaaggaa tttattttca atatcacctt acaaataata 2760

aaaagtcata cagacattgc tttctctttt taattccaag tcatgtacat aaactgactt 2820

ataagtcatc atacaatatt tccataagtt aattccagct ttgagctcca gcttttgttc 2880

cctttagtga gggttaattt cgagcttggc gtaatcatgg tcatagctgt ttcctgtgtg 2940

aaattgttat ccgctcacaa ttccacacaa catacgagcc ggaagcataa agtgtaaagc 3000

ctggggtgcc taatgagtga gctaactcac attaattgcg ttgcgctcac tgcccgcttt 3060

ccagtcggga aacctgtcgt gccagctgca ttaatgaatc ggccaacgcg cggggagagg 3120

cggtttgcgt attgggcgct cttccgcttc ctcgctcact gactcgctgc gctcggtcgt 3180

tcggctgcgg cgagcggtat cagctcactc aaaggcggta atacggttat ccacagaatc 3240

aggggataac gcaggaaaga acatgtgagc aaaaggccag caaaaggcca ggaaccgtaa 3300

aaaggccgcg ttgctggcgt ttttccatag gctccgcccc cctgacgagc atcacaaaaa 3360

tcgacgctca agtcagaggt ggcgaaaccc gacaggacta taaagatacc aggcgtttcc 3420

ccctggaagc tccctcgtgc gctctcctgt tccgaccctg ccgcttaccg gatacctgtc 3480

cgcctttctc ccttcgggaa gcgtggcgct ttctcatagc tcacgctgta ggtatctcag 3540

ttcggtgtag gtcgttcgct ccaagctggg ctgtgtgcac gaaccccccg ttcagcccga 3600

ccgctgcgcc ttatccggta actatcgtct tgagtccaac ccggtaagac acgacttatc 3660

gccactggca gcagccactg gtaacaggat tagcagagcg aggtatgtag gcggtgctac 3720

agagttcttg aagtggtggc ctaactacgg ctacactaga aggacagtat ttggtatctg 3780

cgctctgctg aagccagtta ccttcggaaa aagagttggt agctcttgat ccggcaaaca 3840

aaccaccgct ggtagcggtg gtttttttgt ttgcaagcag cagattacgc gcagaaaaaa 3900

aggatctcaa gaagatcctt tgatcttttc tacggggtct gacgctcagt ggaacgaaaa 3960

ctcacgttaa gggattttgg tcatgagatt atcaaaaagg atcttcacct agatcctttt 4020

aaattaaaaa tgaagtttta aatcaatcta aagtatatat gagtaaactt ggtctgacag 4080

ttaccaatgc ttaatcagtg aggcacctat ctcagcgatc tgtctatttc gttcatccat 4140

agttgcctga ctccccgtcg tgtagataac tacgatacgg gagggcttac catctggccc 4200

cagtgctgca atgataccgc gagacccacg ctcaccggct ccagatttat cagcaataaa 4260

ccagccagcc ggaagggccg agcgcagaag tggtcctgca actttatccg cctccatcca 4320

gtctattaat tgttgccggg aagctagagt aagtagttcg ccagttaata gtttgcgcaa 4380

cgttgttgcc attgctacag gcatcgtggt gtcacgctcg tcgtttggta tggcttcatt 4440

cagctccggt tcccaacgat caaggcgagt tacatgatcc cccatgttgt gcaaaaaagc 4500

ggttagctcc ttcggtcctc cgatcgttgt cagaagtaag ttggccgcag tgttatcact 4560

catggttatg gcagcactgc ataattctct tactgtcatg ccatccgtaa gatgcttttc 4620

tgtgactggt gagtactcaa ccaagtcatt ctgagaatag tgtatgcggc gaccgagttg 4680

ctcttgcccg gcgtcaatac gggataatac cgcgccacat agcagaactt taaaagtgct 4740

catcattgga aaacgttctt cggggcgaaa actctcaagg atcttaccgc tgttgagatc 4800

cagttcgatg taacccactc gtgcacccaa ctgatcttca gcatctttta ctttcaccag 4860

cgtttctggg tgagcaaaaa caggaaggca aaatgccgca aaaaagggaa taagggcgac 4920

acggaaatgt tgaatactca tactcttcct ttttcaatat tattgaagca tttatcaggg 4980

ttattgtctc atgagcggat acatatttga atgtatttag aaaaataaac aaataggggt 5040

tccgcgcaca tttccccgaa aagtgc 5066

<210> 40
<211> 5014
<212> DNA
<213> Artificial

<220>
<223> pBleuScriptSK(-)-6MIE-DsRed2-U100pA sequence in Example 2

<400> 40
cacctgacgc gccctgtagc ggcgcattaa gcgcggcggg tgtggtggtt acgcgcagcg 60

tgaccgctac acttgccagc gccctagcgc ccgctccttt cgctttcttc ccttcctttc 120

tcgccacgtt cgccggcttt ccccgtcaag ctctaaatcg ggggctccct ttagggttcc 180

gatttagtgc tttacggcac ctcgacccca aaaaacttga ttagggtgat ggttcacgta 240

gtgggccatc gccctgatag acggtttttc gccctttgac gttggagtcc acgttcttta 300

atagtggact cttgttccaa actggaacaa cactcaaccc tatctcggtc tattcttttg 360

atttataagg gattttgccg atttcggcct attggttaaa aaatgagctg atttaacaaa 420

aatttaacgc gaattttaac aaaatattaa cgcttacaat ttccattcgc cattcaggct 480

gcgcaactgt tgggaagggc gatcggtgcg ggcctcttcg ctattacgcc agctggcgaa 540

agggggatgt gctgcaaggc gattaagttg ggtaacgcca gggttttccc agtcacgacg 600

ttgtaaaacg acggccagtg aattgtaata cgactcacta tagggcgaat tgggtaccgg 660

gccccccctc gaggtcgaga gttaaagatc agcgggtacc gcggttgaag tatttcctgc 720

ccaggcgaat gagaactcta aaagctcgta acgcggcaac cgagttcctg tttttcccat 780

aaagttaaag atcagcgggt accgcggttg aagtatttcc tgtccaggcg aatgagaact 840

ctaaaagctc gtaacgcggc aatcgcagtt cctgtttttc tcataaattt aaagatcagc 900

gggtaccgcg gttgaagtat ttcctgccca ggcgaatgag aactctaaaa gctcgtaacg 960

cggcaaccgc agttcctgtt tttcttataa agttaaaaat tagcagatat tgcggttgaa 1020

gtctttcctg ttcatgcgaa ttaaaactct aaaaactgct aacgaagcaa ccgagttcct 1080

gtttttctca ttaagttaaa ggttagtggg tactgtggtt ggggtctttc ctacccaggc 1140

taacgagaac cctaaaatct gctaacagag caaccgcagt tcctgttttt ctcataaagt 1200

taaaggtcag tgtgtaccgc ggttacaaca ttttcccctg actaagtcat ttatttcgtg 1260

agaagcgcta acaccaaaac cacattcctg tttcatgatg tgtagcagat gtttttaaaa 1320

aaaaaacatg acaatttatc agtaaagtgt tctttattat cccgccttca accgcaaact 1380

ccgtctttct cataaaaaaa tacaagtcag ccataagaag aaaacctcaa aaaatccaga 1440

ccacaaattc ctgtttttga gtaagatatg acaaaaccct aaatttttgt aagcatcagc 1500

taatttccat tccatatttg tctaaaaggg gtgtatttct acacttgcgg tttaacatta 1560

tacagcgatt ggctccttca tcctcgtcat tttcctgtac atcacacccg ctatagaatt 1620

gtatataagc agaagttaca gccagttcag tgccactttt ctcaagaagt ggctccggag 1680

aacattctca tcacagagat tctttcttat atcgctgcag tctggtaagt caatttttta 1740

actattaata tttaaaattg ctacaaaaat tattttaaat tgaatttaag aatgtttaaa 1800

acaatttaat gatattttca tgcgcataat tgtaatttga taagcacgaa agaatttata 1860

ctgttcacat ttaatcttac agatttgtga cattgattac atcccatcaa ttattggatt 1920

gctggtcgac ggtatcgata agcttgatat cgaattccgc caccatggcc tcctccgaga 1980

acgtcatcac cgagttcatg cgcttcaagg tgcgcatgga gggcaccgtg aacggccacg 2040

agttcgagat cgagggcgag ggcgagggcc gcccctacga gggccacaac accgtgaagc 2100

tgaaggtgac caagggcggc cccctgccct tcgcctggga catcctgtcc ccccagttcc 2160

agtacggctc caaggtgtac gtgaagcacc ccgccgacat ccccgactac aagaagctgt 2220

ccttccccga gggcttcaag tgggagcgcg tgatgaactt cgaggacggc ggcgtggcga 2280

ccgtgaccca ggactcctcc ctgcaggacg gctgcttcat ctacaaggtg aagttcatcg 2340

gcgtgaactt cccctccgac ggccccgtga tgcagaagaa gaccatgggc tgggaggcct 2400

ccaccgagcg cctgtacccc cgcgacggcg tgctgaaggg cgagacccac aaggccctga 2460

agctgaagga cggcggccac tacctggtgg agttcaagtc catctacatg gccaagaagc 2520

ccgtgcagct gcccggctac tactacgtgg acgccaagct ggacatcacc tcccacaacg 2580

aggactacac catcgtggag cagtacgagc gcaccgaggg ccgccaccac ctgttcctgt 2640

agtctagagc ggccgcccct cagaccctac gttgccctca ccttatggca acggacatta 2700

ttaaaataaa aaaattactg aaagagagtc agaaattgtg tcacatgtta ttttattaaa 2760

tcttactgaa ctttgtcctt tgtccttaca aaccttccct atcacaatac gagctccagc 2820

ttttgttccc tttagtgagg gttaatttcg agcttggcgt aatcatggtc atagctgttt 2880

cctgtgtgaa attgttatcc gctcacaatt ccacacaaca tacgagccgg aagcataaag 2940

tgtaaagcct ggggtgccta atgagtgagc taactcacat taattgcgtt gcgctcactg 3000

cccgctttcc agtcgggaaa cctgtcgtgc cagctgcatt aatgaatcgg ccaacgcgcg 3060

gggagaggcg gtttgcgtat tgggcgctct tccgcttcct cgctcactga ctcgctgcgc 3120

tcggtcgttc ggctgcggcg agcggtatca gctcactcaa aggcggtaat acggttatcc 3180

acagaatcag gggataacgc aggaaagaac atgtgagcaa aaggccagca aaaggccagg 3240

aaccgtaaaa aggccgcgtt gctggcgttt ttccataggc tccgcccccc tgacgagcat 3300

cacaaaaatc gacgctcaag tcagaggtgg cgaaacccga caggactata aagataccag 3360

gcgtttcccc ctggaagctc cctcgtgcgc tctcctgttc cgaccctgcc gcttaccgga 3420

tacctgtccg cctttctccc ttcgggaagc gtggcgcttt ctcatagctc acgctgtagg 3480

tatctcagtt cggtgtaggt cgttcgctcc aagctgggct gtgtgcacga accccccgtt 3540

cagcccgacc gctgcgcctt atccggtaac tatcgtcttg agtccaaccc ggtaagacac 3600

gacttatcgc cactggcagc agccactggt aacaggatta gcagagcgag gtatgtaggc 3660

ggtgctacag agttcttgaa gtggtggcct aactacggct acactagaag gacagtattt 3720

ggtatctgcg ctctgctgaa gccagttacc ttcggaaaaa gagttggtag ctcttgatcc 3780

ggcaaacaaa ccaccgctgg tagcggtggt ttttttgttt gcaagcagca gattacgcgc 3840

agaaaaaaag gatctcaaga agatcctttg atcttttcta cggggtctga cgctcagtgg 3900

aacgaaaact cacgttaagg gattttggtc atgagattat caaaaaggat cttcacctag 3960

atccttttaa attaaaaatg aagttttaaa tcaatctaaa gtatatatga gtaaacttgg 4020

tctgacagtt accaatgctt aatcagtgag gcacctatct cagcgatctg tctatttcgt 4080

tcatccatag ttgcctgact ccccgtcgtg tagataacta cgatacggga gggcttacca 4140

tctggcccca gtgctgcaat gataccgcga gacccacgct caccggctcc agatttatca 4200

gcaataaacc agccagccgg aagggccgag cgcagaagtg gtcctgcaac tttatccgcc 4260

tccatccagt ctattaattg ttgccgggaa gctagagtaa gtagttcgcc agttaatagt 4320

ttgcgcaacg ttgttgccat tgctacaggc atcgtggtgt cacgctcgtc gtttggtatg 4380

gcttcattca gctccggttc ccaacgatca aggcgagtta catgatcccc catgttgtgc 4440

aaaaaagcgg ttagctcctt cggtcctccg atcgttgtca gaagtaagtt ggccgcagtg 4500

ttatcactca tggttatggc agcactgcat aattctctta ctgtcatgcc atccgtaaga 4560

tgcttttctg tgactggtga gtactcaacc aagtcattct gagaatagtg tatgcggcga 4620

ccgagttgct cttgcccggc gtcaatacgg gataataccg cgccacatag cagaacttta 4680

aaagtgctca tcattggaaa acgttcttcg gggcgaaaac tctcaaggat cttaccgctg 4740

ttgagatcca gttcgatgta acccactcgt gcacccaact gatcttcagc atcttttact 4800

ttcaccagcg tttctgggtg agcaaaaaca ggaaggcaaa atgccgcaaa aaagggaata 4860

agggcgacac ggaaatgttg aatactcata ctcttccttt ttcaatatta ttgaagcatt 4920

tatcagggtt attgtctcat gagcggatac atatttgaat gtatttagaa aaataaacaa 4980

ataggggttc cgcgcacatt tccccgaaaa gtgc 5014

<210> 41
<211> 3545
<212> DNA
<213> Human herpesvirus 6 (HHV-6)

<400> 41
caagaagtgg ctccggagaa cattctcatc acagagattc tttcttatat cgctgcagtc 60

tggatttgtg acattgatta catcccatca attattggat tgctggattt tvttcccgcc 120

cagaagaaag attgattgca cccgaaacca ccacctggra tcaatctcca aagaaacctc 180

caccttgaat cactcttaaa agaacagtag gcggtgtctg aatttgcatc ttaatataca 240

atggagtcag caaaagatac aacccctact tctatgttca ttctcggaaa accctctgga 300

aacaacatgg aatccaatga ggaacgtatg caaaactacc atcccgatcc agtggtggaa 360

gaatccatca aagaaatatt ggaagagagt ctcaagtgtg atgtgtcctt tgaaagcctg 420

ctttttccag aactggaagc ctttgatctt ttcatcccag agtcttccaa cgacatcgct 480

tccaagaatg tgtcctattc cagtaatgtg gaagaaggag catctgaaga attcaaaact 540

ttggttgctc agtccgttgg cacttgcatc caaagtattg gtgcatcagt gaaggctgcc 600

atgaaacagg aactgtctaa catggaagac gatttaatta actccgctgg cttattaacg 660

ctgcacagat ccatgttgga ggggctggta ttagaacaat taggacagtt gataaatatt 720

aatttactaa gtagtgtttc atctaaattt gtttcaagtt atgcaaaaat gttatctgga 780

aaaaacttgg atttttttaa ttggtgcgaa ccgcgattca ttgtttttgc atgtgataaa 840

tttgatgggt tagtgaaaaa gatggcatca gaatccagag atttgctcat ggatttgaaa 900

gctaatatga acaatcaatt tgttatagcc atcaaaaaca ttttttccaa agcttatgtg 960

gcattagatt gtgaaaaatt gaacatggtt tccacctcat tgttgttgat ggcacacaac 1020

aaagagatgt ccaatcctga catctccaat aaggattttt gtgggcgagt aaatttactt 1080

aaacaggaac tactggaaag tagaaatgag ataatagaaa accatgttaa aaatatgaaa 1140

atgtttcagg aatttgctaa taagcagatg aatcaaatct ttatggataa ctgtgataga 1200

acattcctta aaattcatgt gaattgtaaa aatttaatta ctgccgccaa aaatattagc 1260

actgctgttc tccaaagtat tgttctatgc agcagtgaat tttcatggca atacctcaaa 1320

cctcacagac accagtttaa aattaccata atgaatatga tcacccacgc atgtgaatac 1380

ttagaaacag tttatgatga tactggtttg attaaacctt taacttcctc tgatataatg 1440

gaaggctaca ttgctataaa taaagataga gagtcttcca tctgtgattt aaacatagac 1500

ccttcagaat caatactatt agagttagct gaatttgatg aacatggaaa atattcagag 1560

gaatcttcta tcgaatctat ccatgaagat gatgacaatg tagaatattt aaaatacatg 1620

gaagtacaat ctcctactga taataatact cccaccccat caaagaacaa tgaatcccca 1680

acaagacaaa aacttacaaa catacatgaa aaagatgtag aaaaaatgta tccagatact 1740

ccttctccag atgtgccagg gaaatccaaa gaggcaaaaa cattcattga aaatagcaga 1800

cagataggga aaaaacaaac aagccctaac tgtgtatgta cagcctcagt gacagatctg 1860

ggcggcccta ataactttaa atccattact ggccttgaaa gttgtaaaca cgtcttgata 1920

gaaaaactac ttgaaactca acccgactca gtagttgtag agaccgggtc tgaacaacaa 1980

gacattttgg cttacagccc cgatcaaagg tcacagacaa aagaaaggat tcaggaaaaa 2040

ggttctaact ccaagtgtac cgaaacgctt ccttgtatga cctttaccga ctcagcaact 2100

cctgtcaaaa gtcatgatgc aattcaggat actttaaatc cagaaagtaa aatagacaaa 2160

gaattgggag ctgtagaaag tctagtgaat ctatgtgatg gtttccatga caacccttta 2220

atatcagaaa tgataacatt tggttatgaa actgatcact cagcacctta tgagagtgaa 2280

agcgataaca atgatgaaac tgattacatt gcagactgtg attcaactgt cagaactaat 2340

aacattcata tgaataacac taatgagaat acaccattta gcaagtctct atattcacca 2400

ccagaggtca ctccttctaa ggaaaatgac aaaactgaga aaattgtgcc tgtgactcag 2460

aaatgcaaat ctaaaaagag gacagctaaa aggaaaaatg ttccaattaa accatccaaa 2520

tctaaaaaga ttaagataga taagttacct gaaaccacaa acgtaattgt tataagttca 2580

gagagtgaag atgaagagga tggtaacaat aatattgata gctcaatctt aaaaaaaact 2640

attaaatcag aacctaattc tgaatcgagc agcgagtcag atgactgtac atctgaagat 2700

aattacttac atttgagtga ttatgataaa gtgatcaata atggtcattg taaaagtaaa 2760

ggttttgtat ccccagtgtt taccatccca ataaggtcaa tgccgggtac ccatgatatc 2820

agaaacaaat ttgttcccaa aaaaaattgg ttgtggtttc tgaggaaaac tcacaaagta 2880

gacaattgtg ttatacacag ttctgcaaaa atgaatgtaa aaaatgacag tgatgggaca 2940

gaagccaatc actgcttcat caatcacttt gtccccatta aaatggatga tgaagaatat 3000

aataaggata atgtctcata tacttatagt aaaatacagg atagcaaaac agacttggga 3060

gtcataacac caaccaagaa acttatcaca gaaatggtca tggaaaattt tatggatcta 3120

actgatatca ttaaacacgg catagacaaa cattgccaag atctttccag taaatatact 3180

gttatcacct ctactgcatg tgagaaaaat ttaaatgtga caaattccca aagtcttgta 3240

acagccgaaa ctcaaatatt tgacccgcag ggaacaggaa ataactcccc catactaaat 3300

attatcaatg acacaacatg tcacaatgat gagaacagat gtatggaagg tacaagtaat 3360

gataatgaaa aatgtaccat tagaagtgat tgcaacagtg ataaaatgga agtctttaaa 3420

ttagatggct atccctctga ttatgatcca ttcgaagaaa atgctcaaat ttattagcta 3480

gatgaaacca agatgtaaat tgattagcaa tcattagcat acagatgtgt attatgtaaa 3540

tatgc 3545